# EUROPEAN PATENT APPLICATION

(11) **EP 3 643 697 A1**
(43) Date of publication of application: **29.04.2020**
(21) Application number: 19212327.1
(22) Date of filing: 26.02.2016
(51) Int. Cl.: C07C 39/23, C07C 39/42, A61K 31/05, A61P 19/02, A61P 9/04

(54) **TREATMENT METHOD, COMPOUNDS, AND METHOD OF INCREASING TRPV2 ACTIVITY**

(30) Priority: 27.02.2015 US 201562126167 P
(62) Divisional of application: 16756446.7
(71) Applicant: The Feinstein Institute for Medical Research, Manhasset, NY 11030 (US)
(72) Inventor: GULKO, Percio S., Bronx, NY New York 10471 (US); AL-ABED, Yousef, Dix Hills, NY New York 11746 (US)
(74) Representative: Adamson Jones

(57) **Abstract**

The present invention relates to a compound having the following structure: 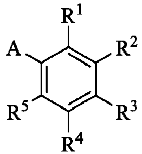 where substituents A and R¹-R⁵ are as defined herein. The compound may be used in a method of treating a subject for a disease or disorder associated with Trpv2 activity. Also disclosed is a composition and a method of increasing Trpv2 activity in a cell or tissue.

## Description

This application claims the benefit of U.S. Provisional Patent Application Serial No. 62/126,167, filed February 27, 2015, which is hereby incorporated by reference in its entirety.

### FIELD OF THE INVENTION

The present invention relates to a treatment method, compounds, and a method of increasing Trpv2 activity.

### BACKGROUND OF THE INVENTION

Rheumatoid arthritis ("RA") affects nearly 1% of the population (Simonsson et al., "The Prevalence of Rheumatoid Arthritis in Sweden," Scand. J. Rheumatol. 28(6):340-343 (1999)) and is associated with reduced quality of living, increased risk for disability and reduced survival (van Zeben et al., "Prognostic Factors in Rheumatoid Arthritis," J. Rheumatol. Suppl. 44:31-33 (1996); Gossec et al., "Prognostic Factors for Remission in Early Rheumatoid Arthritis: A Multiparameter Prospective Study," Ann. Rheum. Dis. 63(6):675-680 (2004); Wolfe et al., "The Mortality of Rheumatoid Arthritis," Arthritis Rheum. 37(4):481-494 (1994)). New and more effective therapies emerged during the past decade significantly improving disease control and quality of living. Yet, disease remission is rarely achieved in RA, underscoring the need for developing more effective therapies. One way of identifying new targets for therapy is to better understand the processes regulating arthritis severity and articular damage, which are major predictors of disease outcome such as the risk of developing deformities and disability.

The hyperplastic synovial tissue in RA, also called pannus, has unique characteristics and, like cancer, invades and destroys cartilage and bone. While the RA synovial tissue behavior is incompletely understood, the joint destruction it mediates correlates with increased disease severity and worse outcome. The fibroblast-like synoviocyte ("FLS") has a central role in the formation of the RA synovial pannus (Bartok et al., "Fibroblast-Like Synoviocytes: Key Effector Cells in Rheumatoid Arthritis," Immunol. Rev. 233(1):233-255 (2010)), and the *in vitro* invasive properties of FLS correlate with radiographic and histologic damage in RA (Tolboom et al., "Invasiveness of Fibroblast-Like Synoviocytes is an Individual Patient Characteristic Associated with the Rate of Joint Destruction in Patients with Rheumatoid Arthritis," Arthritis Rheum. 52(7): 1999-2002 (2005)) and in animal models of arthritis such as pristane-induced arthritis ("PIA") (Laragione et al., "The Arthritis Severity Locus Cia5d is a Novel Genetic Regulator of the Invasive Properties of Synovial Fibroblasts," Arthritis Rheum. 58(8):2296-2306 (2008)). Therefore, understanding the regulation of the invasive properties of FLS has the potential to generate new therapies aimed at reducing articular damage (Laragione et al., "mTOR Regulates the Invasive Properties of Synovial Fibroblasts in Rheumatoid Arthritis," Mol. Med. 16(9-10):352-358 (2010); Gulko, "Contribution of Genetic Studies in Rodent Models of Autoimmune Arthritis to Understanding and Treatment of Rheumatoid Arthritis," Genes Immun. 8(7):523-531 (2007)).

*Trpv2* (Transient receptor potential vanilloid subfamily, type 2 channel) is a non-selective cation channel gene that was detected for the first time in highly invasive FLS from rats with PIA (Laragione et al., "Cia5d Regulates a New Fibroblast-Like Synoviocyte Invasion-Associated Gene Expression Signature," Arthritis Res. Ther. 10(4):R92 (2008)). FLS are known to express other ion channels, including those of the TRP family, such as Trpv1, Trpv3, Trpv4 (Engler et al., "Expression of Transient Receptor Potential Vanilloid 1 (TRPV1) in Synovial Fibroblasts from Patients with Osteoarthritis and Rheumatoid Arthritis," Biochem. Biophys. Res. Commun. 359(4):884-888 (2007); Kochukov et al., "Thermosensitive TRP Ion Channels Mediate Cytosolic Calcium Response in Human Synoviocytes," Am. J. Physiol. Cell Physiol. 291(3):C424-432 (2006)), Trpc1, Trpc5, and Trpm3 (Ciurtin et al., "TRPM3 Channel Stimulated by Pregnenolone Sulphate in Synovial Fibroblasts and Negatively Coupled to Hyaluronan," BMC Musculoskelet. Disord. 11:111 (2010); Xu et al, "TRPC Channel Activation by Extracellular Thioredoxin," Nature 451(7174):69-72 (2008)), but their role in FLS function remains unknown. Trpv2 is related to Trpv1, but has a higher temperature threshold for heat activation (>52°C) (Caterina et al., "A Capsaicin-Receptor Homologue with a High Threshold for Noxious Heat," Nature 398(6726):436-441 (1999)) and has not been implicated in the regulation of pain. Trpv2 is expressed by dorsal root ganglia, lung, placenta, myeloid cells such as macrophages and mast cells, NK, and B cells. Trpv2 was recently implicated in macrophage phagocytosis (Link et al., "TRPV2 has a Pivotal Role in Macrophage Particle Binding and Phagocytosis," Nat. Immunol. 11(3):232-239 (2010)), but other than that its role in human disease remains largely unknown.

The present invention is directed to overcoming deficiencies in the art related to treatment of rheumatoid arthritis and other diseases/disorders associated with Trpv2 activity and/or function, as well as compounds useful in such treatments.

### SUMMARY OF THE INVENTION

One aspect of the present invention relates to a method of treating a subject for a disease or disorder associated with Trpv2 activity. This method involves administering to a subject a compound of formula (Ia) 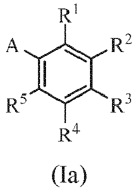 or a stereoisomer, pharmaceutically acceptable salt, oxide, solvate, or ester thereof, where
A is C₆₋₈ cycloalkenyl optionally substituted from 1 to 3 times with substituents independently selected from the group consisting of hydrogen, C₁₋₆ alkyl, and C₂₋₆ alkenyl;
R¹ is halogen, OH, C₁₋₆ alkyl, C₂₋₆ alkenyl, or C₂₋₆ alkynyl, where C₂₋₆ alkenyl is optionally substituted from 1 to 3 times with aryl, and where aryl is optionally substituted from 1 to 3 times with substituents independently selected from the group consisting of hydrogen, OH, and C₁₋₆ alkyl;
R² is hydrogen or C₆₋₈ cycloalkenyl, where C₆₋₈ cycloalkenyl is optionally substituted from 1 to 3 times with substituents independently selected from the group consisting of hydrogen, C₁₋₆ alkyl, and C₂₋₆ alkenyl;
R³ is halogen, OH, C₂₋₆ alkenyl, or C₂₋₆ alkynyl, where C₂₋₆ alkenyl is optionally substituted from 1 to 3 times with aryl, and where aryl is optionally substituted from 1 to 3 times with substituents independently selected from the group consisting of hydrogen, OH, and C₁₋₆ alkyl;
R⁴ is hydrogen or C₆₋₈ cycloalkenyl, where C₆₋₈ cycloalkenyl is optionally substituted from 1 to 3 times with substituents independently selected from the group consisting of hydrogen, C₁₋₆ alkyl, and C₂₋₆ alkenyl; and
R⁵ is halogen, OH, C₁₋₆ alkyl, C₂₋₆ alkenyl, or C₂₋₆ alkynyl, where C₂₋₆ alkenyl is optionally substituted from 1 to 3 times with aryl, and where aryl is optionally substituted from 1 to 3 times with substituents independently selected from the group consisting of hydrogen, OH, and C₁₋₆ alkyl,
   under conditions effective to treat the subject for the disease or disorder associated with Trpv2 activity.

Another aspect of the present invention relates to a compound of formula (Ib) 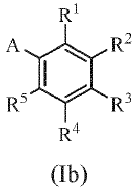 or a stereoisomer, pharmaceutically acceptable salt, oxide, solvate, or ester thereof, where
A is C₆₋₈ cycloalkenyl optionally substituted from 1 to 3 times with substituents independently selected from the group consisting of hydrogen, C₁₋₆ alkyl, and C₂₋₆ alkenyl;
R¹ is OH, C₂₋₆ alkenyl, or C₂₋₆ alkynyl, where C₂₋₆ alkenyl is optionally substituted from 1 to 3 times with aryl, and where aryl is optionally substituted from 1 to 3 times with substituents independently selected from the group consisting of hydrogen, OH, and C₁₋₆ alkyl;
R² is hydrogen or C₆₋₈ cycloalkenyl, where C₆₋₈ cycloalkenyl is optionally substituted from 1 to 3 times with substituents independently selected from the group consisting of hydrogen, C₁₋₆ alkyl, and C₂₋₆ alkenyl;
R³ is halogen, OH, C₂₋₆ alkenyl, or C₂₋₆ alkynyl, where C₂₋₆ alkenyl is optionally substituted from 1 to 3 times with aryl, and where aryl is optionally substituted from 1 to 3 times with substituents independently selected from the group consisting of hydrogen, OH, and C₁₋₆ alkyl;
R⁴ is hydrogen or C₆₋₈ cycloalkenyl, where C₆₋₈ cycloalkenyl is optionally substituted from 1 to 3 times with substituents independently selected from the group consisting of hydrogen, C₁₋₆ alkyl, and C₂₋₆ alkenyl; and
R⁵ is OH, C₂₋₆ alkenyl, and C₂₋₆ alkynyl, where C₂₋₆ alkenyl is optionally substituted from 1 to 3 times with aryl, and where aryl is optionally substituted from 1 to 3 times with substituents independently selected from the group consisting of hydrogen, OH, and C₁₋₆ alkyl;
with the proviso that (i) R³ cannot be F and (ii) when R¹, R³, and R⁵ are OH, R² and R⁴ cannot both be hydrogen.

Another aspect of the present invention relates to a method of increasing Trpv2 activity in a cell or tissue. This method involves providing a compound of formula (Ia) 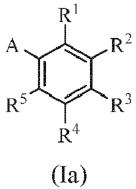 or a stereoisomer, pharmaceutically acceptable salt, oxide, solvate, or ester thereof, where
A is C₆₋₈ cycloalkenyl optionally substituted from 1 to 3 times with substituents independently selected from the group consisting of hydrogen, C₁₋₆ alkyl, and C₂₋₆ alkenyl;
R¹ is halogen, OH, C₁₋₆ alkyl, C₂₋₆ alkenyl, or C₂₋₆ alkynyl, where C₂₋₆ alkenyl is optionally substituted from 1 to 3 times with aryl, and where aryl is optionally substituted from 1 to 3 times with substituents independently selected from the group consisting of hydrogen, OH, and C₁₋₆ alkyl;
R² is hydrogen or C₆₋₈ cycloalkenyl, where C₆₋₈ cycloalkenyl is optionally substituted from 1 to 3 times with substituents independently selected from the group consisting of hydrogen, C₁₋₆ alkyl, and C₂₋₆ alkenyl;
R³ is halogen, OH, C₂₋₆ alkenyl, or C₂₋₆ alkynyl, where C₂₋₆ alkenyl is optionally substituted from 1 to 3 times with aryl, and where aryl is optionally substituted from 1 to 3 times with substituents independently selected from the group consisting of hydrogen, OH, and C₁₋₆ alkyl;
R⁴ is hydrogen or C₆₋₈ cycloalkenyl, where C₆₋₈ cycloalkenyl is optionally substituted from 1 to 3 times with substituents independently selected from the group consisting of hydrogen, C₁₋₆ alkyl, and C₂₋₆ alkenyl; and
R⁵ is halogen, OH, C₁₋₆ alkyl, C₂₋₆ alkenyl, or C₂₋₆ alkynyl, where C₂₋₆ alkenyl is optionally substituted from 1 to 3 times with aryl, and where aryl is optionally substituted from 1 to 3 times with substituents independently selected from the group consisting of hydrogen, OH, and C₁₋₆ alkyl. A cell or tissue is contacted with the compound under conditions effective to increase Trpv2 activity in the cell or tissue.

Increased expression of Trpv2 in highly invasive FLS initially suggested that Trpv2 might have an invasion-inducing effect. However, studies with siRNA and agonists revealed that Trpv2 is in fact a suppressor of cell invasion, inflammatory cell infiltration, angiogenesis, and arthritis severity. The experiments described herein are the first to show Trpv2 agonists used *in vivo.*

The experiments described herein identify a new role for Trpv2 in the regulation of arthritis severity, pannus formation, FLS invasion, and joint damage. The results also suggest a new role for Trpv2 in the regulation of angiogenesis. These new discoveries demonstrate that Trpv2 agonists are a novel strategy for treating diseases and disorders associated with Trpv2 activity. Also disclosed is a new class of drugs for treating diseases and disorders associated with Trpv2 activity, including rheumatoid arthritis and other diseases such as cancers where cell invasion and angiogenesis have a central role in pathogenesis.

### BRIEF DESCRIPTION OF THE DRAWINGS

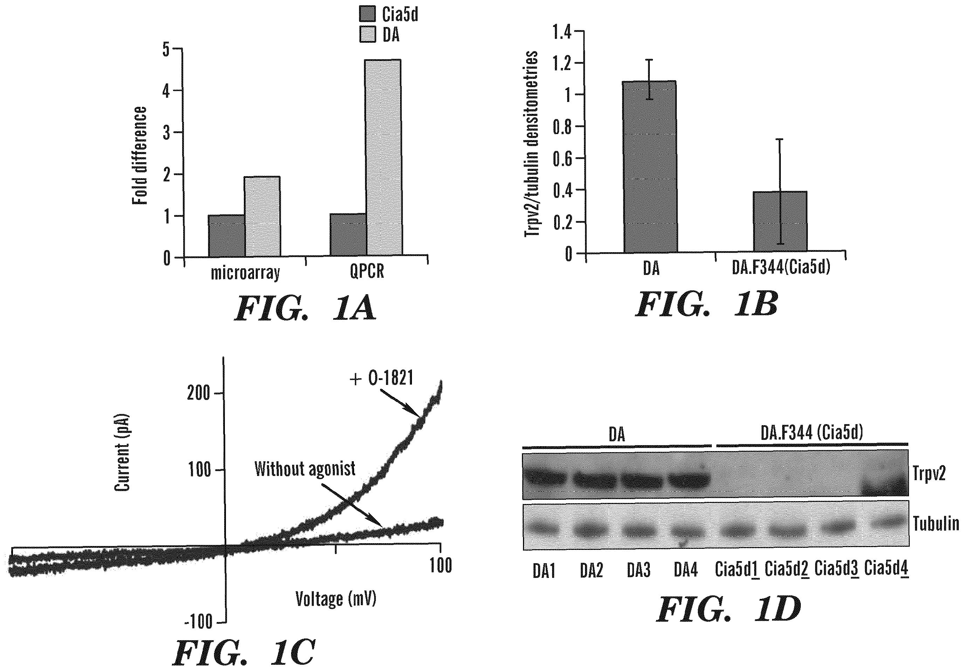

FIGs. 1A-D show that Trpv2 is functional and expressed in increased levels in invasive FLS from DA rats (*i.e*., DA/BklArbNsi, arthritis-susceptible rats). In the graph of FIG. 1A, microarray analysis and QPCR confirmation shows increased mRNA levels of Trpv2 in DA FLS compared with DA.F344(Cia5d) (n=6 per group). FIG. 1B is a graph showing that Trpv2 protein levels were also increased in DA FLS, compared with DA.F344(Cia5d), as shown on densitometries (normalized for tubulin±S.D) from Western blot (FIG. ID). FIG. 1C is a graph showing that FLS obtained from DA rats with PIA were patch-clamped in the absence of agonist, and after perfusion with 30 µM of compound 01821, which induced channel opening. At positive potentials, the current is outward (upward), matching the Trvp2 pattern, thus confirming that FLS have functional Trpv2 channels. Currents are representative of 3-5 cells isolated from 3 different rats with PIA (n = 11 cells total). FIG. ID is a Western blot image of four different DA and four different DA.F344(Cia5d) FLS cell lines (each from a different rat) demonstrating significantly higher Trpv2 protein levels in DA FLS (2.9-fold; densitometries on FIG. 1B).

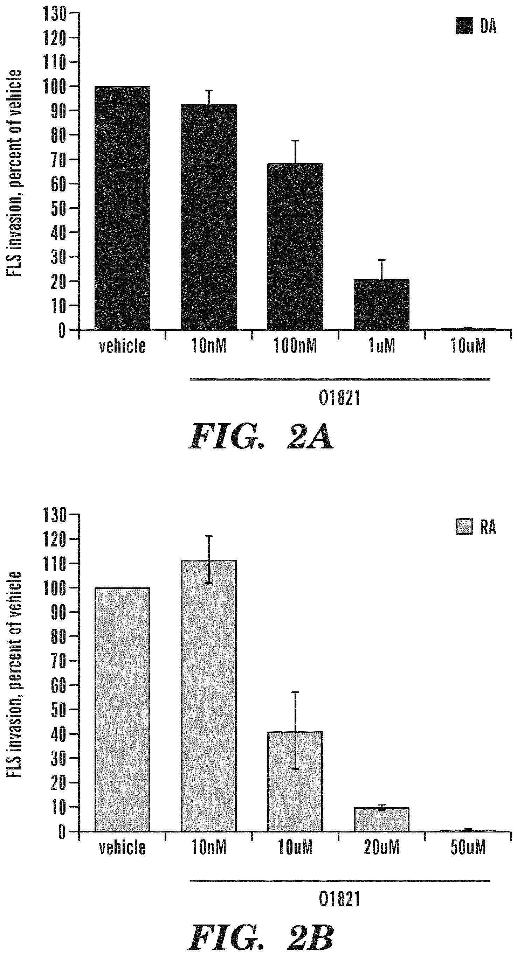

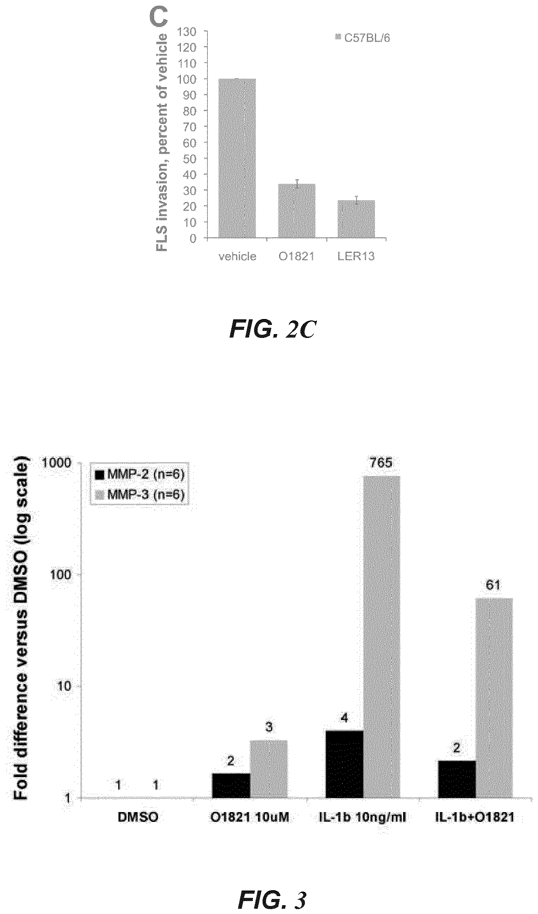

FIGs. 2A-C show that Trpv2 stimulation decreases the invasiveness of FLS from rodents and RA patients. Invasiveness of FLS from both DA (FIG. 2A) and RA (FIG. 2B) treated with the Trvp2 agonist 01821 was significantly reduced in a dose-dependent manner by as much as 100% compared with controls treated with vehicle alone (24h treatment; four different DA FLS cell lines, and seven different RA FLS lines) (mean±S.E.M.; p≤0.001, Rank Sum Test). FIG. 2C is a graph showing that FLS obtained from a single C57BL/6 mouse with KRN serum-induced arthritis had a reduction in invasion of 65% and 75% in the presence of the compound 01821 (10µM) and LER13 (the structure of which is identified as Compound 13 in Table 1 *infra, i.e.,* (1R,1"R,2R,2"S)-6'-ethynyl-5,5"-dimethyl-2,2"-di(prop-1-en-2-yl)-1,1",2,2",3,3",4,4"-octahydro-[1,1':3',1"-terphenyl]-2',4'-diol) (10µM), respectively, compared with control vehicle (one cell line tested in triplicate).
FIG. 3 shows that a Trpv2 agonist decreases IL-1β-induced expression of matrix metalloproteases in RA FLS. FLS derived from synovial tissues from six different patients with RA were stimulated with IL-1β 10ng/ml for 48 hours in the presence or absence of the Trpv2 agonist 01821 (10µM). qPCR demonstrated that IL-1β induced a significant 765-fold increase in MMP-3 and a 4-fold increase in MMP-2 expression. Trpv2 stimulation significantly decreased the IL-1β-induced expression of MMP-2 and MMP-3 by 50% and 90%, respectively (number on each bar is the fold-difference compared with the DMSO treatment control group; data shown in log scale).

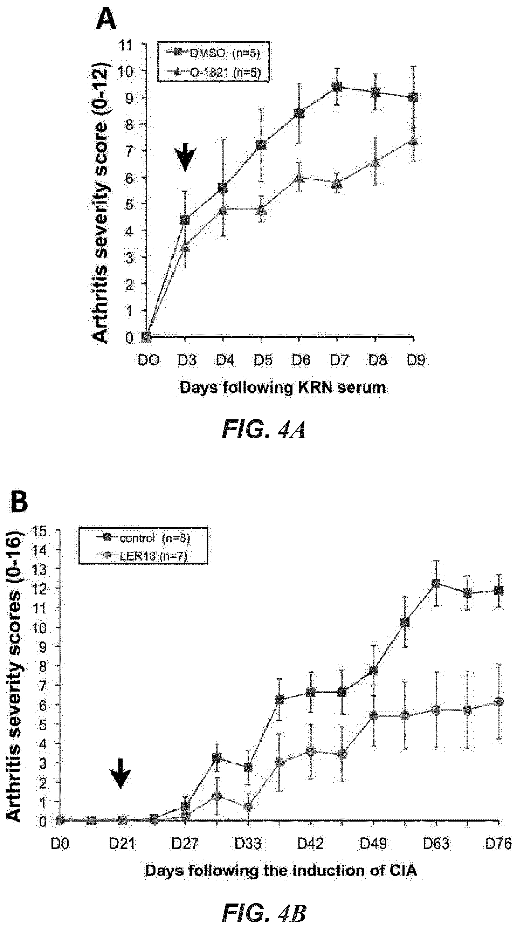

FIGs. 4A-B show that Trpv2 agonists significantly reduced clinical arthritis inflammation and severity *in vivo.* In the graph of FIG. 4A, C57BL/6 mice were injected with KRN arthritogenic serum. Compound 01821 (10mg/Kg BID IP) was started after the onset of arthritis (day 3; D3, black arrow) and continued through day nine (D9). O1821-treated mice had reductions in arthritis severity scores that were evident as early as day five (D5) and reached statistical significance on days seven and eight (D6: P=0.091; D7: P=0.002; D8: P=0.045; mean±SEM; t-test). In the graph of FIG. 4B, DBA1/J mice with collagen-induced arthritis ("CIA") treated with LER13 (*i.e.,* Compound 13 in Table 1 *infra*) (n=8 per treatment group) (treatment started on D10 following the first immunization, black arrow) had a significant reduction in arthritis severity and clinical signs of inflammation compared with the vehicle group. CIA onset is typically around D21 following immunization, and the disease-protecting effect was detected by day 31 and persisted through the end of the arthritis scoring period at day 76 (D76), with a reduction of 50% in mean cumulative arthritis scores (arthritis severity index, ASI; mean±S.D.: control=80±21.8 and LER13=40.7±36.8; p=0.023, t-test), compared with control vehicle.

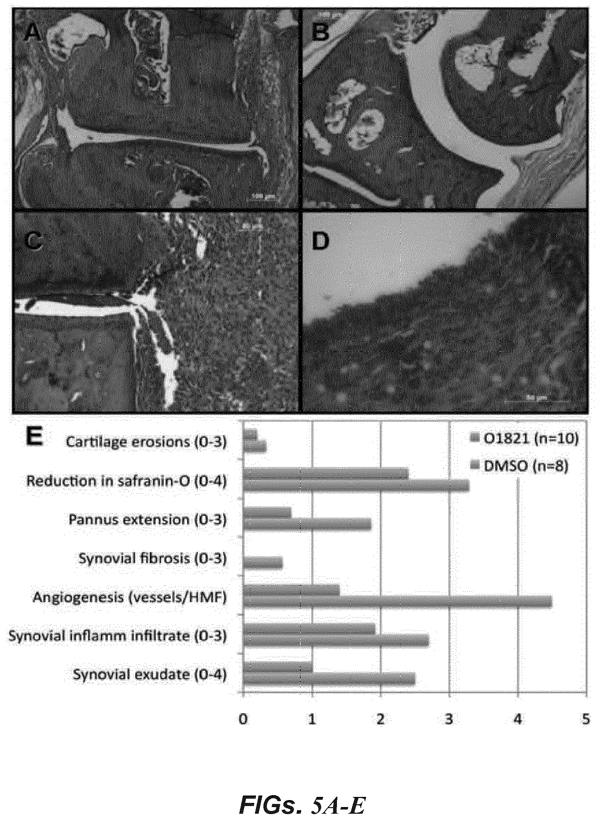

FIGs. 5A-E show that arthritic mice treated with a Trpv2 agonist preserved a normal joint and synovial architecture. In the image of FIG. 5A, C57BL/6 mice with KRN serum transfer-induced arthritis treated with control vehicle developed pronounced ankle synovial hyperplasia and pannus formation. In the image of FIG. 5B, mice treated with the compound 01821 had normal ankle joint and synovial histologies with no pannus formation, and reduced inflammatory infiltration. In the image of FIGs. 5C-D, the synovial tissues from vehicle-treated mice had pronounced inflammation and hyperplasia (H&E staining). FIG. 5E is a graph of results at the end of the arthritis study (D10) where both ankles (thus the higher sample size) were collected and prepared for histology (H&E and Safranin-O for proteoglycan analyses). Slides were scored without knowledge of treatment group (blindly) using a comprehensive histologic scoring system (Brenner et al., "The Non-MHC Quantitative Trait Locus Cia10 Contains a Major Arthritis Gene and Regulates Disease Severity, Pannus Formation and Joint Damage," Arthritis Rheum. 52(1):322-332 (2005), which is hereby incorporated by reference in its entirety). Treatment with 01821 reduced several histologic parameters, including angiogenesis, inflammatory infiltration, pannus formation, and loss of proteoglycan.

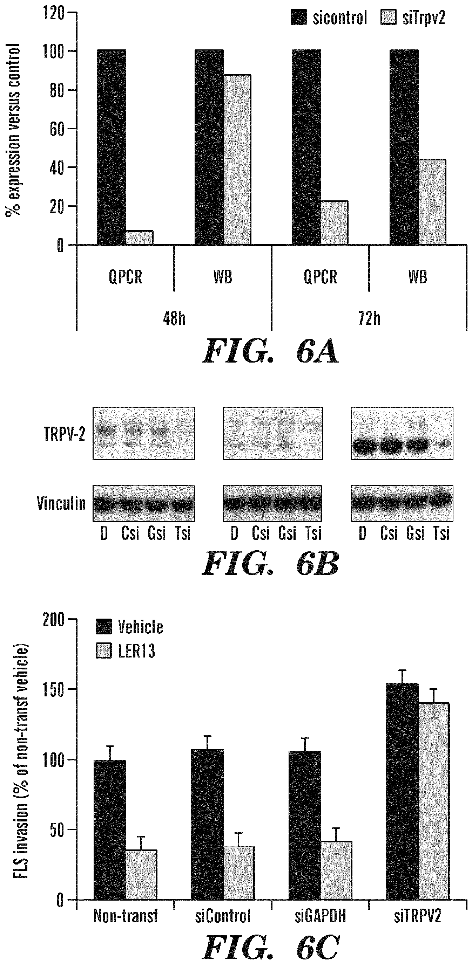

FIGs. 6A-C show that the compound LER13 (*i*.*e*., Compound 13 in Table 1 *infra*) suppresses FLS invasion in a Trpv2-dependent manner. In the graph of FIG. 6A, siRNA targeting Trpv2 achieved an 80% reduction in mRNA levels and a 50% reduction in protein levels 72 hours after transfection (shown as percentage of the control; QPCR=quantitative PCR and WB=Western blot, where levels were normalized for vinculin densitometries). FIG. 6B shows Western blot 72h after Trpv2 siRNA knockdown showing a 50% or greater reduction in protein levels (D=DMSO control; Csi=control siRNA; Gsi=GAPDH siRNA; Tsi=TRPV2 siRNA; each blot was done with FLS from a different DA rat). FIG. 6C is a graph showing that LER13 (10µM) suppresses FLS invasion by 60% in non-transfected (Non-transf), siRNA control (siControl) and in siRNA GAPDH (siGAPDH), but not in siRNA Trpv2 (siTRPV2), demonstrating that LER13 invasion-suppressive activity is dependent on the expression of Trpv2.

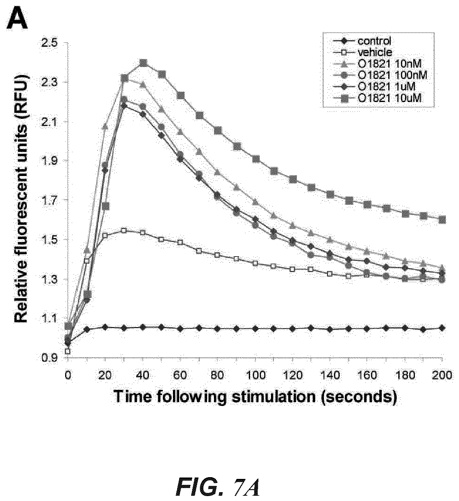

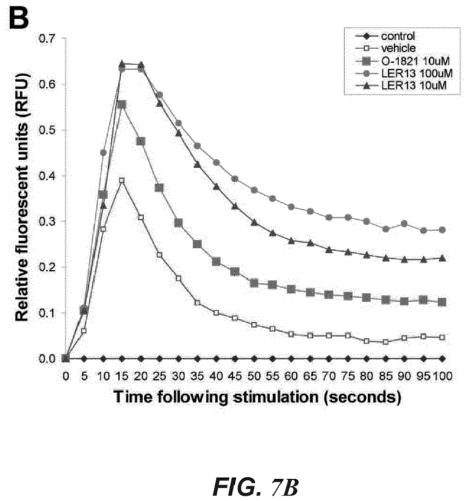

FIGs. 7A-B are graphs showing that Trpv2 agonists 01821 and LER13 (*i*.*e*., Compound 13 in Table 1 *infra*) significantly increased intracellular calcium influx in FLS. Two different DA FLS cell lines were stimulated with increasing concentrations of 01821 (FIG. 7A) and LER13 (FIG. 7B), and induced a significant increase in intracellular calcium influx, compared with untreated controls and vehicle (DMSO). LER13 induced a 30% higher calcium influx compared with 01821.

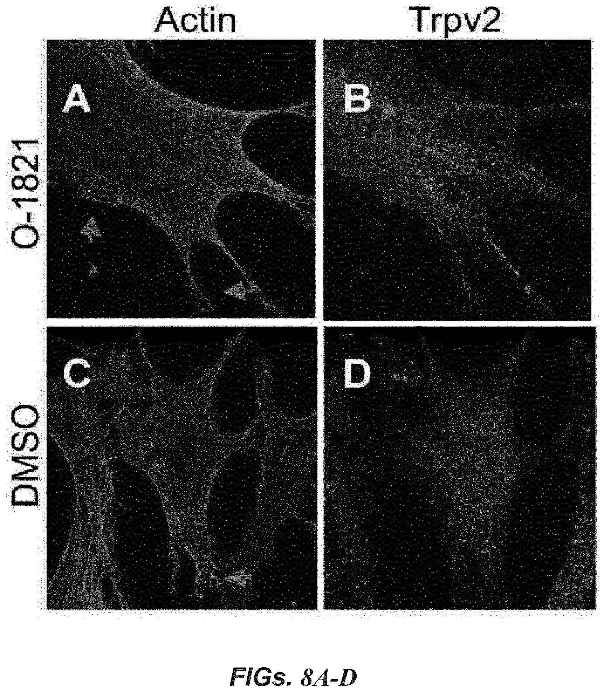

FIGs. 8A-D show Confocal microscopy of Trpv2 distribution in FLS. RA FLS cell lines (n=3) were cultured in complete medium with 10% FBS and starved overnight, then treated with 01821 (1µM, 30 minutes) (FIGs. 8A-B) or DMSO (FIGs. 8C-D), then fixed and stained. RA FLS expressed Trpv2 in the cytoplasm and plasma membrane, including in lamellipodia (grey arrows). However, 01821 treatment did not affect the actin cytoskeleton organization or the number and distribution of lamellipodia. FIGS. 8A, 8C: red=rodhamine phalloidin (actin staining); FIGS 8B, 8D: green=Alexa488 Trpv2. Confocal Microscope Fluoview 300, 600X magnification.

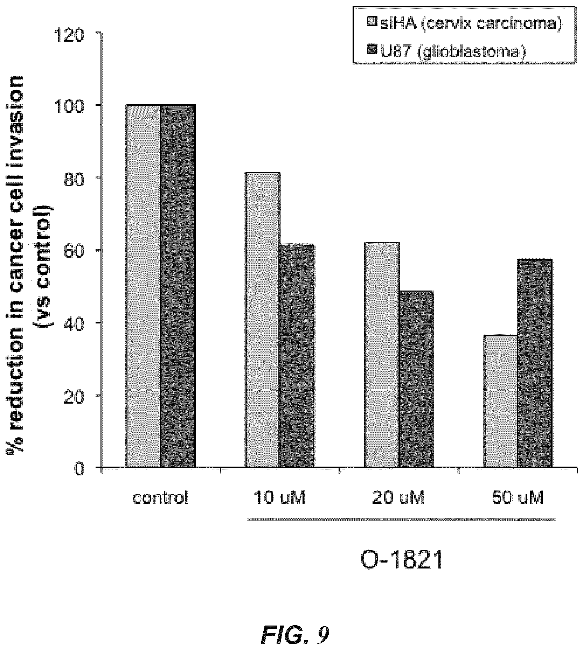

FIG. 9 is a bar graph showing that compound 0-1821 significantly reduces cell invasion in two different cancer cell lines (cervix carcinoma and glioblastoma).

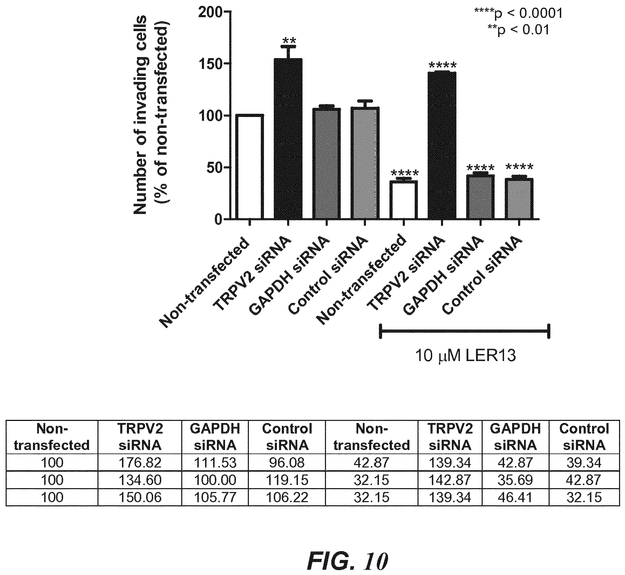

FIG. 10 is a graph showing that Trpv2 expression is required for the LER13-induced suppression of synovial cell invasion (cells treated with siRNA against Trpv2 are not responsive or suppressed by LER13).

### DETAILED DESCRIPTION OF THE INVENTION

One aspect of the present invention relates to a method of treating a subject for a disease or disorder associated with Trpv2 activity. This method involves administering to a subject a compound of formula (Ia) 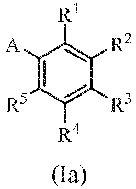 or a stereoisomer, pharmaceutically acceptable salt, oxide, solvate, or ester thereof, where
A is C₆₋₈ cycloalkenyl optionally substituted from 1 to 3 times with substituents independently selected from the group consisting of hydrogen, C₁₋₆ alkyl, and C₂₋₆ alkenyl;
R¹ is halogen, OH, C₁₋₆ alkyl, C₂₋₆ alkenyl, or C₂₋₆ alkynyl, where C₂₋₆ alkenyl is optionally substituted from 1 to 3 times with aryl, and where aryl is optionally substituted from 1 to 3 times with substituents independently selected from the group consisting of hydrogen, OH, and C₁₋₆ alkyl;
R² is hydrogen or C₆₋₈ cycloalkenyl, where C₆₋₈ cycloalkenyl is optionally substituted from 1 to 3 times with substituents independently selected from the group consisting of hydrogen, C₁₋₆ alkyl, and C₂₋₆ alkenyl;
R³ is halogen, OH, C₂₋₆ alkenyl, or C₂₋₆ alkynyl, where C₂₋₆ alkenyl is optionally substituted from 1 to 3 times with aryl, and where aryl is optionally substituted from 1 to 3 times with substituents independently selected from the group consisting of hydrogen, OH, and C₁₋₆ alkyl;
R⁴ is hydrogen or C₆₋₈ cycloalkenyl, where C₆₋₈ cycloalkenyl is optionally substituted from 1 to 3 times with substituents independently selected from the group consisting of hydrogen, C₁₋₆ alkyl, and C₂₋₆ alkenyl; and
R⁵ is halogen, OH, C₁₋₆ alkyl, C₂₋₆ alkenyl, or C₂₋₆ alkynyl, where C₂₋₆ alkenyl is optionally substituted from 1 to 3 times with aryl, and where aryl is optionally substituted from 1 to 3 times with substituents independently selected from the group consisting of hydrogen, OH, and C₁₋₆ alkyl,
   under conditions effective to treat the subject for the disease or disorder associated with Trpv2 activity.

As used herein, the term "halogen" means fluoro, chloro, bromo, or iodo.

The term "optionally substituted" indicates that a group may have a substituent at each substitutable atom of the group (including more than one substituent on a single atom), and the identity of each substituent is independent of the others.

The term "substituted" means that one or more hydrogen on a designated atom is replaced with a selection from the indicated group, provided that the designated atom's normal valency is not exceeded. "Unsubstituted" atoms bear all of the hydrogen atoms dictated by their valency. When a substituent is oxo (*i.e.,* =O), then 2 hydrogens on the atom are replaced. Combinations of substituents and/or variables are permissible only if such combinations result in stable compounds. By "stable compound" it is meant a compound that is sufficiently robust to survive isolation to a useful degree of purity from a reaction mixture, and formulation into an efficacious therapeutic agent.

The term "alkyl" means an aliphatic hydrocarbon group which may be straight or branched having about 1 to about 6 carbon atoms in the chain (or the number of carbons designated by "Cₙ₋ₙ", where n-n is the numerical range of carbon atoms). Branched means that one or more lower alkyl groups such as methyl, ethyl or propyl are attached to a linear alkyl chain. Exemplary alkyl groups include methyl, ethyl, n-propyl, i-propyl, n-butyl, t-butyl, n-pentyl, and 3-pentyl.

The term "alkenyl" means an aliphatic hydrocarbon group containing a carbon-carbon double bond and which may be straight or branched having about 2 to about 6 carbon atoms in the chain, or 2 to about 4 carbon atoms in the chain. Branched means that one or more lower alkyl groups such as methyl, ethyl, or propyl are attached to a linear alkenyl chain. Exemplary alkenyl groups include ethenyl, propenyl, n-butenyl, and i-butenyl.

The term "alkynyl" means an aliphatic hydrocarbon group containing a carbon-carbon triple bond and which may be straight or branched having about 2 to about 6 carbon atoms in the chain, or 2 to about 4 carbon atoms in the chain. Branched means that one or more lower alkyl groups such as methyl, ethyl, or propyl are attached to a linear alkynyl chain. Exemplary alkynyl groups include ethynyl, propynyl, n-butynyl, 2-butynyl, 3-methylbutynyl, and n-pentynyl.

The term "aryl" refers to an aromatic monocyclic or polycyclic ring system containing from 6 to 19 carbon atoms, where the ring system may be optionally substituted. Aryl groups of the present invention include, but are not limited to, groups such as phenyl, naphthyl, azulenyl, phenanthrenyl, anthracenyl, fluorenyl, pyrenyl, triphenylenyl, chrysenyl, and naphthacenyl. The term "monocyclic" indicates a molecular structure having one ring. The term "polycyclic" indicates a molecular structure having two or more rings, including, but not limited to, fused, bridged, or spiro rings.

The term "cycloalkenyl" refers to a non-aromatic, unsaturated, mono- or polycyclic ring system of about 6 to about 8 carbon atoms, which includes at least one double bond. Exemplary cycloalkenyl groups include, without limitation, cyclopropenyl, cyclobutenyl, cyclopentenyl, cyclohexenyl, and the like.

The term "compound(s) of the invention" and equivalent expressions, are meant to embrace compounds herein described, which expression includes the prodrugs, the pharmaceutically acceptable salts, the oxides, and the solvates, e.g. hydrates, where the context so permits.

The term "method of treating" means amelioration or relief from the symptoms and/or effects associated with the diseases or disorders described herein.

Compounds described herein may contain one or more asymmetric centers and may thus give rise to enantiomers, diastereomers, and other stereoisomeric forms. Each chiral center may be defined, in terms of absolute stereochemistry, as (R)- or (S)-. The present invention is meant to include all such possible isomers, as well as mixtures thereof, including racemic and optically pure forms. Optically active (R)- and (S)-, (-)- and (+)-, or (D)- and (L)-isomers may be prepared using chiral synthons or chiral reagents, or resolved using conventional techniques. When the compounds described herein contain olefinic double bonds or other centers of geometric asymmetry, and unless specified otherwise, it is intended that the compounds include both E and Z geometric isomers. Likewise, all tautomeric forms are also intended to be included.

As would be understood by a person of ordinary skill in the art, the recitation of "a compound" is intended to include salts, solvates, oxides, and inclusion complexes of that compound as well as any stereoisomeric form, or a mixture of any such forms of that compound in any ratio. Thus, in accordance with some embodiments of the invention, a compound as described herein, including in the contexts of pharmaceutical compositions, methods of treatment, and compounds *per se*, is provided as the salt form.

The term "solvate" refers to a compound in the solid state, where molecules of a suitable solvent are incorporated in the crystal lattice. A suitable solvent for therapeutic administration is physiologically tolerable at the dosage administered. Examples of suitable solvents for therapeutic administration are ethanol and water. When water is the solvent, the solvate is referred to as a hydrate. In general, solvates are formed by dissolving the compound in the appropriate solvent and isolating the solvate by cooling or using an antisolvent. The solvate is typically dried or azeotroped under ambient conditions.

Inclusion complexes are described in Remington, The Science and Practice of Pharmacy, 19th Ed. 1:176-177 (1995), which is hereby incorporated by reference in its entirety. The most commonly employed inclusion complexes are those with cyclodextrins, and all cyclodextrin complexes, natural and synthetic, are specifically encompassed by the present invention.

The term "pharmaceutically acceptable salt" refers to salts prepared from pharmaceutically acceptable non-toxic acids or bases including inorganic acids and bases and organic acids and bases.

The configuration of any carbon-carbon double bond appearing herein is selected for convenience only and is not necessarily intended to designate a particular configuration; thus a carbon-carbon double bond depicted arbitrarily herein as E may be Z, E, or a mixture of the two in any proportion.

The term "pharmaceutically acceptable" means it is, within the scope of sound medical judgment, suitable for use in contact with the cells of humans and lower animals without undue toxicity, irritation, allergic response and the like, and are commensurate with a reasonable benefit/risk ratio.

Administering of compounds and/or pharmaceutical compositions to a subject may involve administering therapeutically effective amounts, which means an amount of compound effective in treating the stated conditions and/or disorders in a subject. Such amounts generally vary according to a number of factors well within the purview of ordinarily skilled artisans. These include, without limitation: the particular subject, as well as its age, weight, height, general physical condition, and medical history, the particular compound used, as well as the carrier in which it is formulated and the route of administration selected for it; and, the nature and severity of the condition being treated.

Administering typically involves administering pharmaceutically acceptable dosage forms, which means dosage forms of compounds described herein, and includes, for example, tablets, dragees, powders, elixirs, syrups, liquid preparations, including suspensions, sprays, inhalants tablets, lozenges, emulsions, solutions, granules, capsules, and suppositories, as well as liquid preparations for injections, including liposome preparations. Techniques and formulations generally may be found in Remington's Pharmaceutical Sciences, Mack Publishing Co., Easton, Pa., latest edition, which is hereby incorporated by reference in its entirety.

Administering may be carried out orally, topically, transdermally, parenterally, subcutaneously, intravenously, intramuscularly, intraperitoneally, by intranasal instillation, by intracavitary or intravesical instillation, intraocularly, intraarterially, intralesionally, or by application to mucous membranes. Compounds may be administered alone or with suitable pharmaceutical carriers, and can be in solid or liquid form, such as tablets, capsules, powders, solutions, suspensions, or emulsions.

Diseases or disorders amenable to the treatment method of the present invention include, without limitation, rheumatoid arthritis, psoriasis, psoriatic arthritis, inflammatory diseases, asthma, cancer, diabetic retinopathy, cardiomyopathy, heart failure, and congestive heart failure. In one particular embodiment of this aspect of the present invention, the treatment method is carried out to treat rheumatoid arthritis.

In one embodiment, the compound used in the method of treating a subject for a disease or disorder associated with Trpv2 activity is a compound of formula (IIa) 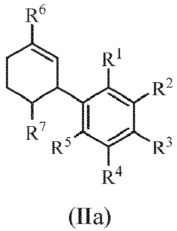 or a stereoisomer, pharmaceutically acceptable salt, oxide, solvate, or ester thereof, where
R¹ is halogen, OH, C₁₋₆ alkyl, C₂₋₆ alkynyl, or 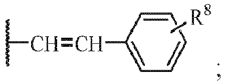
R² is hydrogen or 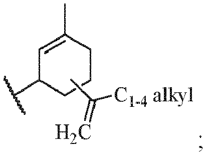
R³ is halogen, OH, 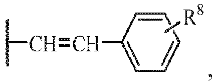 or C₂₋₆ alkynyl;
R⁴ is hydrogen or 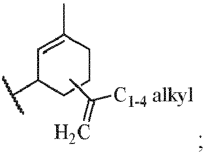
R⁵ is halogen, OH, C₁₋₆ alkyl, C₂₋₆ alkynyl, or 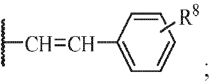
R⁶ is hydrogen or C₁₋₆ alkyl;
R⁷ is 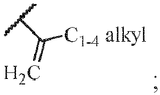 and
R⁸ is hydrogen, OH, or C₁₋₆ alkyl.

In another embodiment, in the compound of formula (IIa)
halogen is F or Cl;
C₁₋₆ alkyl is methyl;
C₁₋₄ alkyl is methyl;
C₂₋₆ alkynyl is C₂ alkynyl;
R⁷ is 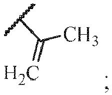 and
R⁸ is OH.

In yet another embodiment, the compound of formula (Ia) (or formula (IIa)) is selected from the group consisting of:

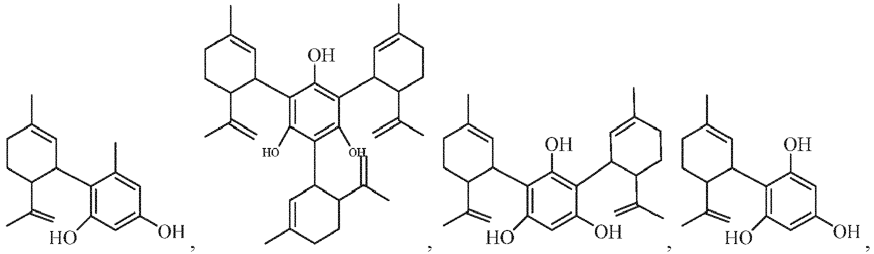

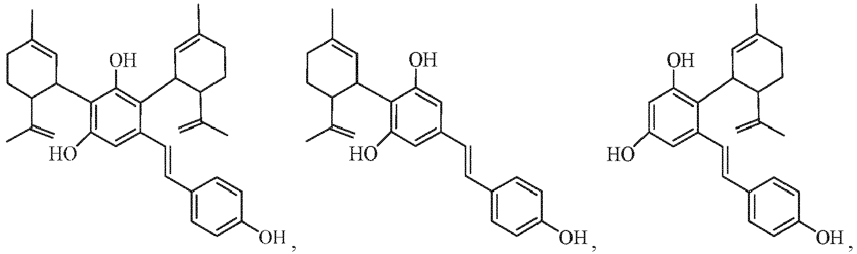

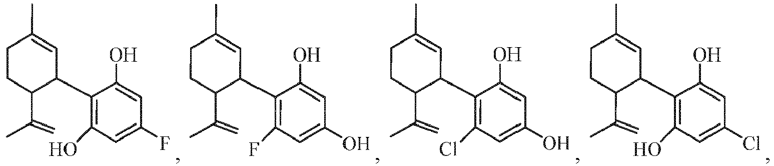

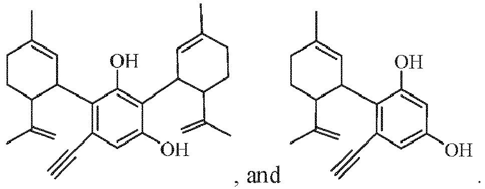

In yet another embodiment, the compound of formula (Ia) (or formula (IIa)) is 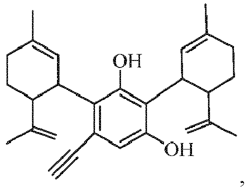 also referred to herein as LER13 (*i.e.,* Compound 13 in Table 1 *infra*).

In carrying out this aspect of the present invention, suitable subjects to be treated include mammals, such as a human.

A second aspect of the present invention relates to a compound of formula (Ib) 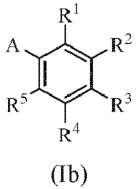 or a stereoisomer, pharmaceutically acceptable salt, oxide, solvate, or ester thereof, where
A is C₆₋₈ cycloalkenyl optionally substituted from 1 to 3 times with substituents independently selected from the group consisting of hydrogen, C₁₋₆ alkyl, and C₂₋₆ alkenyl;
R¹ is OH, C₂₋₆ alkenyl, or C₂₋₆ alkynyl, where C₂₋₆ alkenyl is optionally substituted from 1 to 3 times with aryl, and where aryl is optionally substituted from 1 to 3 times with substituents independently selected from the group consisting of hydrogen, OH, and C₁₋₆ alkyl;
R² is hydrogen or C₆₋₈ cycloalkenyl, where C₆₋₈ cycloalkenyl is optionally substituted from 1 to 3 times with substituents independently selected from the group consisting of hydrogen, C₁₋₆ alkyl, and C₂₋₆ alkenyl;
R³ is halogen, OH, C₂₋₆ alkenyl, or C₂₋₆ alkynyl, where C₂₋₆ alkenyl is optionally substituted from 1 to 3 times with aryl, and where aryl is optionally substituted from 1 to 3 times with substituents independently selected from the group consisting of hydrogen, OH, and C₁₋₆ alkyl;
R⁴ is hydrogen or C₆₋₈ cycloalkenyl, where C₆₋₈ cycloalkenyl is optionally substituted from 1 to 3 times with substituents independently selected from the group consisting of hydrogen, C₁₋₆ alkyl, and C₂₋₆ alkenyl; and
R⁵ is OH, C₂₋₆ alkenyl, and C₂₋₆ alkynyl, where C₂₋₆ alkenyl is optionally substituted from 1 to 3 times with aryl, and where aryl is optionally substituted from 1 to 3 times with substituents independently selected from the group consisting of hydrogen, OH, and C₁₋₆ alkyl;
with the proviso that (i) R³ cannot be F and (ii) when R¹, R³, and R⁵ are OH, R² and R⁴ cannot both be hydrogen.

Compounds of formula (Ia) (and IIa, discussed *infra*) can be prepared *e.g.,* by reacting (+)-*cis*-*p*-mentha-2,8-dien-1-ol **(1)** with analogues of resorcinol **(2)** as shown in Scheme 1 below.

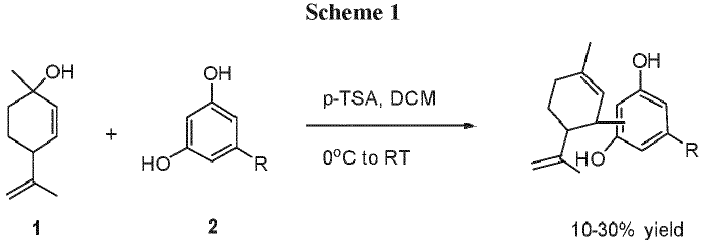

The compounds of formula (Ia) (and the other compounds described herein made by the above process) can be isolated and purified in a known manner, for example, by subjecting the residue after distillation of the solvent to partition, extraction, re-precipitation, recrystallization, or another purification method or combination of purification methods.

In one embodiment, the compound of this aspect of the present invention has a structure of formula (IIb) 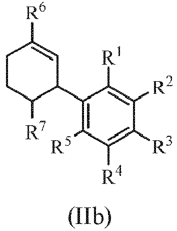 or a stereoisomer, pharmaceutically acceptable salt, oxide, solvate, or ester thereof, where
R¹ and R⁵ are independently OH, C₂₋₆ alkynyl, or 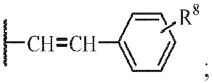
R² and R⁴ are independently hydrogen or 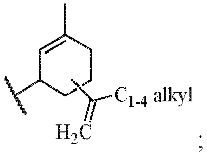
R³ is Cl, OH, 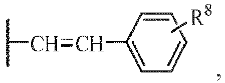 or C₂₋₆ alkynyl;
R⁶ is hydrogen or C₁₋₆ alkyl;
R⁷ is 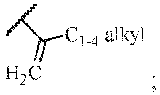 and
R⁸ is hydrogen, OH, or C₁₋₆ alkyl,
with the proviso that when R¹, R³, and R⁵ are OH, R² and R⁴ cannot both be hydrogen.

In another embodiment, the compound has a structure of formula (IIb) where C₂₋₆ alkynyl is C₂ alkynyl;
R⁷ is 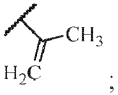 and
R⁸ is OH.

In another embodiment, the compound of formula (Ib) (or formula (IIb)) is selected from the group consisting of:

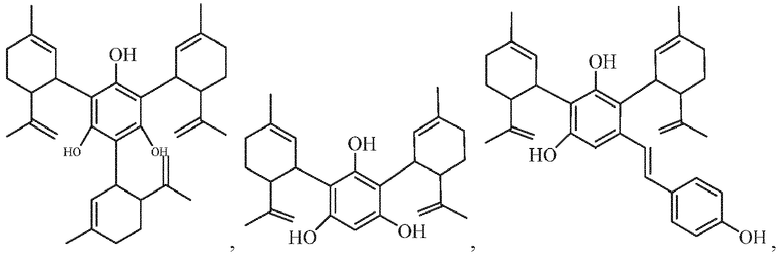

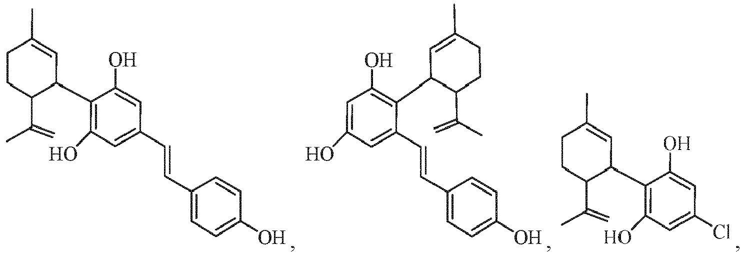

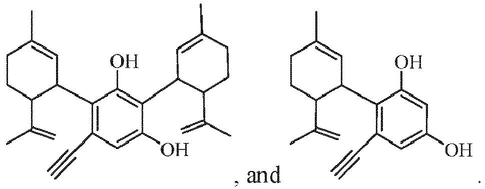

In yet another embodiment, the compound of formula (Ib) (or formula (IIb)) is 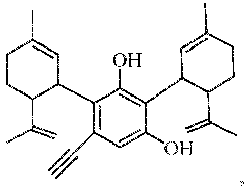 also referred to herein as LER13 (*i.e.,* Compound 13 in Table 1 *infra*).

A further aspect of the present invention is directed to a composition comprising a compound of the present invention and a carrier.

In one embodiment, the carrier is a pharmaceutically-acceptable carrier, and the composition is a pharmaceutical composition. The term "pharmaceutical composition" means a composition comprising a compound of the present invention and at least one component comprising pharmaceutically acceptable carriers, diluents, adjuvants, excipients, or vehicles, such as preserving agents, fillers, disintegrating agents, wetting agents, emulsifying agents, suspending agents, sweetening agents, flavoring agents, perfuming agents, antibacterial agents, antifungal agents, lubricating agents and dispensing agents, depending on the nature of the mode of administration and dosage forms. The term "pharmaceutically acceptable carrier" is used to mean any carrier, diluent, adjuvant, excipient, or vehicle, as described herein. Examples of suspending agents include ethoxylated isostearyl alcohols, polyoxyethylene sorbitol and sorbitan esters, microcrystalline cellulose, aluminum metahydroxide, bentonite, agar-agar and tragacanth, or mixtures of these substances. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride, and the like. Prolonged absorption of the injectable pharmaceutical form can be brought about by the use of agents delaying absorption, for example, aluminum monosterate and gelatin. Examples of suitable carriers, diluents, solvents, or vehicles include water, ethanol, polyols, suitable mixtures thereof, vegetable oils (such as olive oil), and injectable organic esters such as ethyl oleate. Examples of excipients include lactose, milk sugar, sodium citrate, calcium carbonate, and dicalcium phosphate. Examples of disintegrating agents include starch, alginic acids, and certain complex silicates. Examples of lubricants include magnesium stearate, sodium lauryl sulphate, talc, as well as high molecular weight polyethylene glycols.

Another aspect of the present invention relates to a method of increasing Trpv2 activity in a cell or tissue. This method involves providing a compound of formula (Ia) 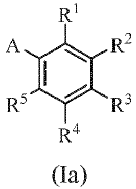 or a stereoisomer, pharmaceutically acceptable salt, oxide, solvate, or ester thereof, where
A is C₆₋₈ cycloalkenyl optionally substituted from 1 to 3 times with substituents independently selected from the group consisting of hydrogen, C₁₋₆ alkyl, and C₂₋₆ alkenyl;
R¹ is halogen, OH, C₁₋₆ alkyl, C₂₋₆ alkenyl, or C₂₋₆ alkynyl, where C₂₋₆ alkenyl is optionally substituted from 1 to 3 times with aryl, and where aryl is optionally substituted from 1 to 3 times with substituents independently selected from the group consisting of hydrogen, OH, and C₁₋₆ alkyl;
R² is hydrogen or C₆₋₈ cycloalkenyl, where C₆₋₈ cycloalkenyl is optionally substituted from 1 to 3 times with substituents independently selected from the group consisting of hydrogen, C₁₋₆ alkyl, and C₂₋₆ alkenyl;
R³ is halogen, OH, C₂₋₆ alkenyl, or C₂₋₆ alkynyl, where C₂₋₆ alkenyl is optionally substituted from 1 to 3 times with aryl, and where aryl is optionally substituted from 1 to 3 times with substituents independently selected from the group consisting of hydrogen, OH, and C₁₋₆ alkyl;
R⁴ is hydrogen or C₆₋₈ cycloalkenyl, where C₆₋₈ cycloalkenyl is optionally substituted from 1 to 3 times with substituents independently selected from the group consisting of hydrogen, C₁₋₆ alkyl, and C₂₋₆ alkenyl; and
R⁵ is halogen, OH, C₁₋₆ alkyl, C₂₋₆ alkenyl, or C₂₋₆ alkynyl, where C₂₋₆ alkenyl is optionally substituted from 1 to 3 times with aryl, and where aryl is optionally substituted from 1 to 3 times with substituents independently selected from the group consisting of hydrogen, OH, and C₁₋₆ alkyl. A cell or tissue is contacted with the compound under conditions effective to increase Trpv2 activity in the cell or tissue.

In one embodiment of this aspect of the present invention, contacting is carried out *in vitro,* such as in a sample. *In vitro* methods may be carried out to test the activity of certain compounds and/or pharmaceutical compositions against cells in, *e.g.,* a solution or a tissue sample, for their ability to modulate Trpv2 activity. In an alternative embodiment, contacting is carried out *in vivo* in an animal or patient or subject.

Modulating (*e.g*., increasing) Trpv2 activity may be accomplished in a cell or tissue by any mechanism of action.

### EXAMPLES

### Example 1 - Synovial Tissue Collection from Rats with Pristane-Induced Arthritis (PIA) and Mice with KRN Serum-Induced Arthritis

Eight to 10 week-old inbred DA (DA/BklArbNsi, arthritis-susceptible) rats received 150 µl of pristane (2,6,10,14-tetramethylpentadecane) (MP Bio, Solon, OH) by intradermal injection (Vingsbo et al., "Pristane-Induced Arthritis in Rats: A New Model for Rheumatoid Arthritis with a Chronic Disease Course Influenced by Both Major Histocompatibility Complex and Non-Major Histocompatibility Complex Genes," Am. J. Pathol. 149(5): 1675-1683 (1996); Brenner et al., "The Non-MHC Quantitative Trait Locus Cia10 Contains a Major Arthritis Gene and Regulates Disease Severity, Pannus Formation and Joint Damage," Arthritis Rheum. 52(1):322-332 (2005), which are hereby incorporated by reference in their entirety). On day 21 post-pristane injection (a time-point when all DA rats have arthritis), animals were euthanized and synovial tissues collected from the ankle joints for FLS isolation. Synovial tissues from C57BL/6 mice (Jackson Laboratories) with KRN serum-induced arthritis were collected at the end of the arthritis observation period (day 10). All experiments involving animals were reviewed and approved by the Feinstein Institute Institutional Animal Care and Use Committee (IACUC).

### Example 2 - RA Patients and Synovial Tissues

Human synovial tissues were obtained from RA patients undergoing elective orthopedic surgery. All patients met the American College of Rheumatology criteria for RA (Arnett et al, "The American Rheumatism Association 1987 Revised Criteria for the Classification of Rheumatoid Arthritis," Arthritis Rheum. 31(3):315-324 (1988), which is hereby incorporated by reference in its entirety). Informed consent was obtained from all participating subjects under an Institutional Review Board-approved protocol through the Feinstein Institute's Tissue Donation Program.

### Example 3 - Isolation and Culture of FLS

FLS were obtained as previously described (Laragione et al., "The Arthritis Severity Locus Cia5d is a Novel Genetic Regulator of the Invasive Properties of Synovial Fibroblasts," Arthritis Rheum. 58(8):2296-2306 (2008), which is hereby incorporated by reference in its entirety). Briefly, freshly obtained synovial tissues were minced and incubated with a solution containing DNase (0.15 mg/ml), hyaluronidase type I-S (0.15 mg/ml), and collagenase type IA (1 mg/ml) (Sigma, St.Louis, MO) in DMEM (Invitrogen, Carlsbad, CA) for 1 hour at 37°C. Cells were washed and re-suspended in complete media containing DMEM supplemented with 10% FBS (Invitrogen), glutamine (300 ng/ml), amphotericin B (250 µg/ml) (Sigma), and gentamicin (20 ng/ml) (Invitrogen). After overnight culture, non-adherent cells were removed and adherent cells cultured. All experiments were performed with FLS after passage four (>95% FLS purity).

### Example 4 - Invasion Assay

The *in vitro* invasiveness of FLS was assayed in a transwell system using Matrigel-coated inserts (BD Biosciences, Franklin Lakes, NJ), as previously described (Tolboom et al., "Invasiveness of Fibroblast-Like Synoviocytes is an Individual Patient Characteristic Associated with the Rate of Joint Destruction in Patients with Rheumatoid Arthritis," Arthritis Rheum. 52(7): 1999-2002 (2005); Laragione et al., "The Arthritis Severity Locus Cia5d is a Novel Genetic Regulator of the Invasive Properties of Synovial Fibroblasts," Arthritis Rheum. 58(8):2296-2306 (2008), which are hereby incorporated by reference in their entirety). Briefly, 70-80% confluent cells were harvested by trypsin-EDTA digestion, and re-suspended at 2.0 x 10⁴ cells in 500 µl of serum-free DMEM. Cells were placed in the upper compartment of the Matrigel-coated inserts. Where indicated, 01821, LER13, or vehicle was added to the upper chamber. The lower compartment was filled with complete media and the plates were incubated at 37°C for 24 hours. After 24 hours the upper surface of the insert was wiped with cotton-swabs to remove non-invading cells and the Matrigel layer. The opposite side of the insert was stained with Crystal Violet (Sigma) and the total number of cells that invaded through Matrigel counted at 100X magnification. Experiments were done in duplicate.

### Example 5 - Trpv2 Agonists

The Trpv2 agonist 01821 (Cayman Chemicals) (Qin et al., "TRPV2 is Activated by Cannabidiol and Mediates CGRP Release in Cultured Rat Dorsal Root Ganglion Neurons," J. Neurosci. 28(24):6231-6238 (2008), which is hereby incorporated by reference in its entirety) is a synthetic cannabinoid that stimulates Trpv2, but does not stimulate Trpv1 or the cannabinoid receptors (Qin et al., "TRPV2 is Activated by Cannabidiol and Mediates CGRP Release in Cultured Rat Dorsal Root Ganglion Neurons," J. Neurosci. 28(24):6231-6238 (2008); Offertaler et al., "Selective Ligands and Cellular Effectors of a G Protein-Coupled Endothelial Cannabinoid Receptor," Mol. Pharmacol. 63(3):699-705 (2003), which are hereby incorporated by reference in their entirety). A focused library was synthesized and screened around 01821, and LER13 was identified as a novel and potent Trpv2 agonist. *See,* Table 1 (*infra*).

### Example 6 - siRNA Knock-Down

Dharmacon SMARTpool siRNA targeting Trpv2, Gapdh, or a non-coding control were purchased from Thermo Scientific (Lafayette, CO) and transfected into DA FLS according to the manufacturer's instructions. Cells were then incubated at 37°C for 72 hours prior to initiating the invasion assays. Knock-down was confirmed with qPCR and Western blot.

### Example 7 - MMP-2 and MMP-3 qPCR Studies

The qPCR conditions used have been previously described (Laragione et al., "Cia5d Regulates a New Fibroblast-Like Synoviocyte Invasion-Associated Gene Expression Signature," Arthritis Res. Ther. 10(4):R92 (2008), which is hereby incorporated by reference in its entirety). Briefly, total RNA (200 ng) from each sample was reverse transcribed using Superscript III (Invitrogen). MMP-2 (forward-CGGTTTTCTCGAATCCATGA (SEQ ID NO: 1); reverse-GGTATCCATCGCCATGCT (SEQ ID NO: 2) and MMP-3 (forward-CCAGGTGTGGAGTTCCTGA (SEQ ID NO: 3); reverse-GCATCTTTTGGCAAATCTGG (SEQ ID NO: 4)) primers were designed based on the Universal ProbeLibrary (Roche, Indianapolis, IN). Reactions were prepared with Absolute Blue qPCR Mix (Thermo Fisher, Waltham, MA), and run in duplicate on a LightCycler 480 thermocycler (Roche) using Relative Quantitative Software (Roche). *Ct* (threshold cycle) values were adjusted for GAPDH in each sample (Δ*Ct*). Fold differences were calculated with the 2^{-ΔΔ*Ct*} method (Livak et al., "Analysis of Relative Gene Expression Data Using Real-Time Quantitative PCR and the 2(-Delta Delta C(T)) Method," Methods 25(4):402-408 (2001), which is hereby incorporated by reference in its entirety).

### Example 8 - Intracellular Calcium Influx

Intracellular calcium influx was measured using the Fluo-4 NW calcium assay kit (Molecular Probes/Invitrogen) according to the manufacturer's instructions, as previously described (Laragione et al., "CXCL10 and its Receptor CXCR3 Regulate Synovial Fibroblast Invasion in Rheumatoid Arthritis," Arthritis Rheum. 63(11):3274-3283 (2011), which is hereby incorporated by reference in its entirety). Briefly, DA FLS were plated at a density of 4,500 cells/well in a 96-well plate (100 µl volume of complete media per well). After cell adhesion the media was changed to serum-free media for overnight culture. Serum-free media was then replaced with 50 µl of dye loading solution, followed by incubation at 37°C for 30 minutes and a baseline fluorimetric reading. Then, 01821, LER13, or vehicle were added to their respective wells at different concentrations and fluorescence read immediately in a Fluoroskan Ascent Microplate Fluorometer (Thermo Scientific) with settings appropriate for excitation at 494 nm and emission at 516 nm.

### Example 9 - Single Cell Patch Clamp Studies

FLS obtained from DA rats with PIA were plated in culture medium onto glass coverslips and allowed to adhere at 37°C. Recordings were performed in the whole-cell configuration of the patch-clamp technique at room temperature using an EPC10-USB amplifier (HEKA instruments, Bellmore, NY). Patch pipettes had a resistance of 2-4 MΩ when filled with a solution containing 45 mM CsCl, 100 mM CsF, 5 mM EGTA, 10 mM HEPES, and 5 mM glucose, pH 7.4. The bath solution contained 132 mM NaCl, 5.4 mM KCl, 1.8 mM CaCl₂, 0.8 mM MgCl₂, 10 mM HEPES, and 10 mM glucose, at pH 7.4. 01821-activated currents were elicited with a 600 ms ramp from -100 to +100 mV from a holding potential of -107 mV. Addition of 01821 was accomplished by complete bath exchange.

### Example 10 - Immunofluorescence and Confocal Microscopy

Immunofluorescence was performed as previously reported (Laragione et al., "mTOR Regulates the Invasive Properties of Synovial Fibroblasts in Rheumatoid Arthritis," Mol. Med. 16(9-10):352-358 (2010); Chan et al., "The GTPase Rac Regulates the Proliferation and Invasion of Fibroblast-Like Synoviocytes from Rheumatoid Arthritis Patients," Mol. Med. 13(5-6):297-304 (2007), which are hereby incorporated by reference in their entirety). Briefly, FLS were cultured on coverslips to 10-20% confluence, starved overnight and then pre-treated for 30 minutes, 60 minutes or 24h with either DMSO or 01821, in the presence or absence of IL-1β 10-25 ng/ml or PDGF 50 ng/ml in complete media or in serum-free media. Cells were then fixed with 4% formaldehyde for 15 minutes at room temperature and permeabilized with PBS/Triton X-100 0.1% for 5 minutes. Non-specific binding was blocked with 5% nonfat milk. A rabbit anti-Trpv2 (Santa Cruz Biotechnology) was used as primary antibody, and a Alexa Fluor 488 (green) donkey anti-rabbit IgG (Invitrogen) used as secondary antibody. Alexa Fluor 594 (red) Phalloidin (Invitrogen) was used to stain the actin filament. The stained cells were mounted on a glass slide. A Zeiss Axiovert 200 M fluorescent microscope was used for visualization with the appropriate filters, with Zeiss Axioversion 4.7 software. Immunofluorescence microscopy was used for cell cytoskeleton scoring as previously described (Laragione et al., "CXCL10 and its Receptor CXCR3 Regulate Synovial Fibroblast Invasion in Rheumatoid Arthritis," Arthritis Rheum. 63(11):3274-3283 (2011), which is hereby incorporated by reference in its entirety). An Olympus FluoView 300 was used for confocal microscopy with a 600X magnification.

### Example 11 - MTT Assay

4x10⁴ FLS per well were plated in triplicate in 96-well plates in 100 µl of complete media. Cells were allowed to adhere for 24 hours. Media was then changed and 01821, LER13, or vehicle was added at the same concentrations used for the invasion studies. After 24 hours (same duration of the invasion experiments) viable cells were determined by the colorimetric MTT kit (Millipore) according to the manufacturer's instructions.

### Example 12 - Mice and in vivo Testing of Trpv2 Agonists

### KRN Serum Transfer-Induced Arthritis

Seven to eight week-old C57BL/6 male mice (Jackson Laboratories) received 200 µl of KRN arthritogenic serum (Ji et al, "Genetic Influences on the End-Stage Effector Phase of Arthritis," J. Exp. Med. 194(3):321-330 (2001), which is hereby incorporated by reference in its entirety) (KBN TCR transgenic mice were a kind gift from Christophe Benoist and have been intercrossed with NOD at the Feinstein Institute to generate KRN mice and the arthritogenic serum) intraperitoneal (IP) on day zero (D0) and day 2 (D2). After the onset of clear ankle arthritis (day 3; D3), mice were started on 01821 10 mg/Kg IP BID or control vehicle (DMSO in saline solution). Arthritis severity was scored according to the system reported by Jirholt et al., "Genetic Linkage Analysis of Collagen-Induced Arthritis in the Mouse," Eur. J. Immunol. 28(10):3321-3328 (1998), which is hereby incorporated by reference in its entirety, which includes a number of involved joints and a degree of inflammation (swelling/redness) in a scale of 0-12 per mouse per day.

### Collagen-Induced Arthritis (CIA)

CIA was induced in 8-week-old male DBA1/J mice (Jackson Laboratories) using a standard protocol (Courtenay et al., "Immunisation Against Heterologous Type II Collagen Induces Arthritis in Mice," Nature 283(5748):666-668 (1980); Nagler-Anderson et al., "Suppression of Type II Collagen-Induced Arthritis by Intragastric Administration of Soluble Type II Collagen," Proc. Natl. Acad. Sci. U.S.A. 83(19):7443-7446 (1986), which are hereby incorporated by reference in their entirety). Briefly, bovine type II collagen (Chondrex, Redmond, WA) was dissolved overnight in acetic acid 0.1 N and then homogenized at 1:1 with Complete Freund's Adjuvant (CFA, Difco, Detroit, MI). Anesthetized mice were injected intradermally with 100 µg of type II collagen emulsified in CFA in the tail. The new Trpv2 agonist LER13 (10 mg/Kg) or control vehicle (PBS 50%, PEG300 40%, PG 5%, PS80 5%) was started on day 10 (D10) and administered intra-peritoneally every-other-day (from D10 until day 55). On day 21 (D21), mice received a booster injection of 100 µg of type II collagen in incomplete Freund's adjuvant. Clinical signs of arthritis were evident on day 24 (D24).

Arthritis severity in CIA mice was scored using a modified scoring system based on Jirholt et al., "Genetic Linkage Analysis of Collagen-Induced Arthritis in the Mouse," Eur. J. Immunol. 28(10):3321-3328 (1998), which is hereby incorporated by reference in its entirety. Briefly, the modified scoring system ranged from 0-16 per mouse per day and included a functional component of disease severity (non-weight bearing) in the maximum score of 4 per paw. The arthritis severity index (ASI), which is the sum of all scores obtained during the observation period, was used to compare treatment groups as it provides a comprehensive assessment of arthritis severity over time, and correlates with histologic damage (Brenner et al., "The Non-MHC Quantitative Trait Locus Cia10 Contains a Major Arthritis Gene and Regulates Disease Severity, Pannus Formation and Joint Damage," Arthritis Rheum. 52(1):322-332 (2005); Brenner et al., "The Non-MHC Quantitative Trait Locus Cia5 Contains Three Major Arthritis Genes that Differentially Regulate Disease Severity, Pannus Formation, and Joint Damage in Collagen- and Pristane-Induced Arthritis," J. Immunol. 174(12):7894-7903 (2005), which are hereby incorporated by reference in their entirety).

### Example 13 - Histology Scoring

Mice were euthanized at the end of the *in vivo* arthritis experiments (76 days), ankles fixed in formalin, decalcified, and embedded in paraffin for H&E and Safranin-O staining. Histology slides were scored without knowledge of treatment and according to a comprehensive scoring system previously reported that includes parameters for synovial hyperplasia, inflammatory cell infiltration, and bone and cartilage erosions (Brenner et al., "The Non-MHC Quantitative Trait Locus Cia10 Contains a Major Arthritis Gene and Regulates Disease Severity, Pannus Formation and Joint Damage," Arthritis Rheum. 52(1):322-332 (2005), which is hereby incorporated by reference in its entirety).

### Example 14 - Statistics

Means were compared with the Student's t-test or one-way ANOVA with a pairwise multiple comparison procedure (Holm-Sidak method) using SigmaStat 3.0 (SPSS, Chicago, IL).

### Example 15 - General Procedure for the Synthesis of Compounds

To a stirred mixture of analogues of resorcinol **2** (1 equiv.) and *p*-TSA (0.15 equiv.) in DCM (0.03-0.5 M) at 0°C, under nitrogen atmosphere, was added a solution of (+)*-cis-p*-mentha-2,8-dien-1-ol (**1,** 1.1 equiv.) in DCM. *See* Scheme 1 *supra.* Following the addition, the reaction temperature was raised to room temperature and stirring was continued for 1-3 hours. The reaction was quenched by the addition of saturated sodium bicarbonate solution, the organic layer was separated, and the aqueous phase was extracted with DCM. The combined organic layer was washed with brine, dried (MgSO₄), and the solvent was removed under reduced pressure. The residue was chromatographed on silica gel to afford the products. (Yield 10%-30%).

### Example 16 - Results

### Trpv2 is Expressed in Increased Levels in Highly Invasive FLS

In microarray studies leading up to this study, the transcriptome of highly invasive FLS obtained from DA (severe and erosive arthritis) and of minimally invasive FLS from DA.F344(Cia5d) (mild and non-erosive arthritis) rat strains was analyzed. An expression signature was identified that included genes implicated in cancer-related phenotypes such as invasion and metastasis (Laragione et al., "mTOR Regulates the Invasive Properties of Synovial Fibroblasts in Rheumatoid Arthritis," Mol. Med. 16(9-10):352-358 (2010); Laragione et al., "Cia5d Regulates a New Fibroblast-Like Synoviocyte Invasion-Associated Gene Expression Signature," Arthritis Res. Ther. 10(4):R92 (2008), which are hereby incorporated by reference in their entirety). One of the most significantly differentially expressed genes was Trpv2. Trpv2 had a 1.9-fold increased expression in highly invasive DA FLS, compared with the minimally invasive FLS from DA.F344(Cia5d) FLS (P=0.0075, FIG. 1A), which initially suggested that this gene could have an invasion-favoring function. Levels of Trpv2 were confirmed with qPCR (4.68-fold; P=0.012, FIG. 1A) and Western blot (2.9-fold, FIGs. 1B and ID).

### Single Cell Patch Clamp Studies Confirmed that Trpv2 is Functional in FLS

At negative potentials, the current was inward (downward in the plot, FIG. 1C) and reverses at 0 mV, while at positive potentials, the current is outward (upward in the plot, FIG. 1C), matching the Trvp2 pattern (Mihara et al., "Involvement of TRPV2 Activation in Intestinal Movement Through Nitric Oxide Production in Mice," J. Neurosci. 30(49): 16536-16544 (2010), which is hereby incorporated by reference in its entirety), and confirming that the Trpv2 channel is not only expressed, but also functional in FLS and opened by 01821 (FIG. 1C).

### Trpv2 Stimulation Increased Intracellular Calcium Influx in FLS

Trpv2 stimulation is known to increase intracellular calcium influx (Caterina et al., "A Capsaicin-Receptor Homologue with a High Threshold for Noxious Heat," Nature 398(6726):436-441 (1999); Iwata et al., "Dominant-Negative Inhibition of Ca2+ Influx Via TRPV2 Ameliorates Muscular Dystrophy in Animal Models," Hum. Mol. Genet. 18(5):824-834 (2009), which are hereby incorporated by reference in their entirety). Therefore, two different DA FLS cells lines were stimulated in duplicate with increasing concentrations of the Trpv2 agonist 01821 (FIG. 7A). 01821 induced a significant dose-dependent increase in intracellular FLS calcium influx, which was more pronounced and more sustained in the highest concentration (10 µM).

### 01821 Significantly Decreased Rodent and RA FLS Invasion

Overnight treatment with 01821 significantly reduced FLS invasion in a dose-dependent manner by 80% at 1 µM, and by 99.4% at 10 µM in DA cells (FIG. 2A). 01821 also reduced RA FLS invasion by 90% at 20 µM and by 99.4% at 50 µM concentrations compared with control vehicle (P≤0.001; FIG. 2B). 01821 also reduced invasion of FLS obtained from a C57BL/6 with KRN serum-induced arthritis (FIG. 2C).

The possibility of cell mortality was examined using the MTT assay over a 24-hour period (same duration of the invasion experiments) and similar numbers of dead cells were identified in the 01821 and control vehicle groups. These observations indicated that 01821 was not toxic to FLS, and that the reduced invasion effect was not explained by increased cell mortality.

These findings demonstrated that Trpv2 was a new suppressor of FLS invasion, and that its increased expression in DA represents a failed attempt at reducing invasion, perhaps due to insufficient levels of endogenous agonists or activators, or reduced signaling activity mediated by the channel.

### Trpv2 is Diffusely Expressed in FLS, Including Within Lamellipodia

Confocal microscopy studies of three DA and three RA FLS lines revealed diffuse cytoplasmic and plasma membrane distribution of Trpv2 (FIGs. 8A-D). Trpv2 was also expressed in lamellipodia (FIGs. 8A and 8C, grey arrows), which are cell protrusions required for FLS invasion, raising the possibility of a localized effect on the production of proteases involved in cell invasion.

### 01821 Does Not Affect FLS Morphology or the Cell Distribution of Trpv2

Stimulation with 01821 (10 µM) for 30 minutes to 24 hours had no significant effect on actin cytoskeleton rearrangements, number or location of lamellipodia, or other cell morphology characteristics (FIG. 8A) in cells stimulated with either PDGF or IL-1β in the presence or absence of 10% FBS. 01821 also did not significantly change the distribution of Trpv2 in FLS (FIG. 8B).

### Trpv2 Stimulation Reduces IL-1β-Induced Expression of MMP-2 and MMP-3 in RA FLS

Treatment of RA FLS with 01821 significantly reduced the IL-1β-induced expression of MMP-2 by 45.6% (P=0.02, n=6 RA; FIG. 3) and MMP-3 by 92% (P<0.001, n=6 RA; FIG. 3).

### The 01821-Induced Suppression of FLS Invasion Did Not Involve Akt, Erk or p65 NFκB

Akt, Erk, and NFκB have been implicated in the regulation of FLS invasion. Akt and Erk had been suggested to mediate Trpv2 activity in experiments with endothelial cells (Offertaler et al., "Selective Ligands and Cellular Effectors of a G Protein-Coupled Endothelial Cannabinoid Receptor," Mol. Pharmacol. 63(3):699-705 (2003), which is hereby incorporated by reference in its entirety) and glioma cells (Nabissi et al., "TRPV2 Channel Negatively Controls Glioma Cell Proliferation and Resistance to Fas-Induced Apoptosis in ERK-Dependent Manner," Carcinogenesis 31(5):794-803 (2010), which is hereby incorporated by reference in its entirety) that were unrelated to invasion, and the ankyrin domains in Trpv2 could potentially interact and interfere with NFκB activity (McCleverty et al., "Crystal Structure of the Human TRPV2 Channel Ankyrin Repeat Domain," Protein Sci. 15(9):2201-2206 (2006), which is hereby incorporated by reference in its entirety). Therefore, the Trpv2-dependent effect of 01821 was considered to involve one of these cell signaling mediators. However, 01821 did not affect FLS levels of phosphorylated Akt or phosphorylated Erk1/2. 01821 did not change p65 NFκB activation based on nuclear location of p65 on immunofluorescence analyses (5, 10, 30, and 60 minutes, and 24 hours).

### 01821 Reduced Disease Severity, Clinical Signs of Inflammation and Histologic Damage in Established KRN Serum Transfer Model of Arthritis

C57BL/6 mice with KRN serum transfer-induced arthritis were started with 01821 10 mg/Kg IP BID or control vehicle (DMSO in saline solution) after the onset of arthritis on day three. By day five (D5), a small difference in arthritis severity was already detected and reached statistical significance on days seven and eight (D7 and D8) (FIG. 4A). The arthritis severity indices (ASI), which are the sum of all scores obtained during the observation period, were compared as they provide a more comprehensive assessment of arthritis over time, and correlate with histologic damage (Brenner et al., "The Non-MHC Quantitative Trait Locus Cia10 Contains a Major Arthritis Gene and Regulates Disease Severity, Pannus Formation and Joint Damage," Arthritis Rheum. 52(1):322-332 (2005); Brenner et al., "The Non-MHC Quantitative Trait Locus Cia5 Contains Three Major Arthritis Genes that Differentially Regulate Disease Severity, Pannus Formation, and Joint Damage in Collagen- and Pristane-Induced Arthritis," J. Immunol. 174(12):7894-7903 (2005), which are hereby incorporated by reference in their entirety). O1821-treated mice had a significantly (30%) lower ASI, compared with control vehicle-treated mice (ASI mean±S.D.: O1821=38.8±6.3 and DMSO control vehicle=53.2±9.1; P=0.02, t-test).

01821 was studied in the KRN serum transfer model over a short period of time (nine days), but that was enough time to detect reduced synovial pannus formation, reduced synovial fibrosis, and preservation of cartilage and proteoglycan staining (Safranin-O), compared with control vehicle (FIG. 5 panels A-E). O1821-treated mice also had reduced synovial tissue inflammatory infiltration, reduced synovial fluid exudates, and marked reduction in angiogenesis (FIGs. 5B and 5E). These observations indicated that in addition to regulating the invasive properties of FLS and MMP expression, Trpv2 is involved in the regulation of both angiogenesis and the influx of inflammatory cells into the synovial tissues. These are new discoveries as Trpv2 had not been previously implicated in these processes.

### A New and More Potent Trpv2 Agonist, LER13, Significantly Reduces FLS Invasion, and Disease Severity in CIA

A focused library was synthesized and screened around 01821 for new Trpv2 agonists. The data are provided in Table 1 below. These compounds were tested for their ability to increase intracellular calcium influx. The five most potent compounds were then tested for their ability to suppress FLS invasion *in vitro,* and arthritis *in vivo.* LER13 induced a nearly 30% higher calcium influx in FLS, compared with 01821 (FIG. 7B), and significantly reduced FLS invasion by 65% both in cells from a mouse with KRN serum-induced arthritis (FIG. 2C) and rats with PIA (FIG. 6C). siRNA knock-down of Trpv2 (FIGs. 6A-B) rendered FLS non-responsive to the invasion-suppressive effect of LER13 (FIG. 6C), demonstrating that the compound functions in a Trpv2-dependent manner. In fact, Trpv2 knock-down increased FLS invasiveness by nearly 60%, further supporting a role for this gene in invasion suppression (FIG. 6C).

**Table 1. Synthesized and Screened Focused Library of Compounds**

| **Cpd.** | **Structure** | **Calcium influx** | **FLS invasion** | **Arthritis severity** |
|---|---|---|---|---|
| 1 | 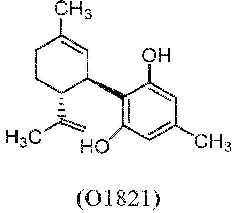 | increased | Decreased by 30% | Protective KRN |
| | (1'R,2'R)-4,5'-dimethyl-2'-(prop-1-en-2-yl)-1',2',3',4'-tetrahydro-[1,1'-biphenyl]-2,6-diol | | | |
| 2 | 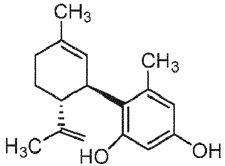 | increased (like DMSO, but less than O1821) | | |
| | (1'R,2'R)-5',6-dimethyl-2'-(prop-1-en-2-yl)-1',2',3',4'-tetrahydro-[1,1'-biphenyl]-2,4-diol | | | |
| 3 | 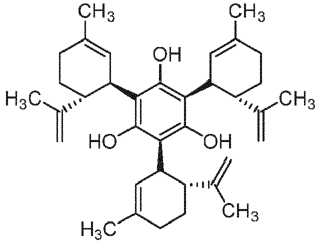 | increased (20% greater than O1821) | | |
| | (1R,1"S,2S,2"R)-5,5"-dimethyl-5'-((1R,6R)-3-methyl-6-(prop-1-en-2-yl)cyclohex-2-en-1-yl)-2,2"-di(prop-1-en-2-yl)-1,1",2,2",3,3",4,4"-octahydro-[1,1':3',1"-terphenyl]-2',4',6'-triol | | | |
| 4 | 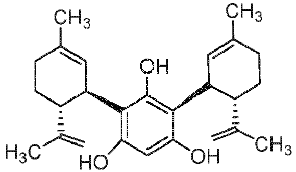 | increased (25% less than O1821) | | |
| | (1R,1"R,2R,2"S)-5,5"-dimethyl-2,2"-di(prop-1-en-2-yl)-1,1",2,2",3,3",4,4"-octahydro-[1,1':3',1"-terphenyl]-2',4',6'-triol | | | |
| 5 | 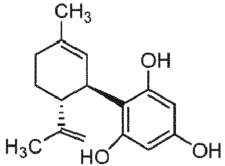 | increased (25% less than O1821) | | |
| | (1'R,2'R)-5'-methyl-2'-(prop-1-en-2-yl)-1',2',3',4'-tetrahydro-[1,1'-biphenyl]-2,4,6-triol | | | |
| 6 | 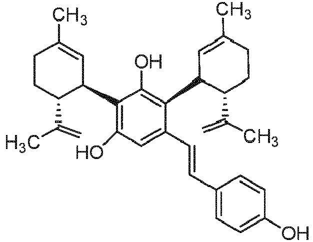 | increased (similar to O1821) | | |
| | (1R,1"R,2S,2"R)-6'-((E)-4-hydroxystyryl)-5,5"-dimethyl-2,2"-di(prop-1-en-2-yl)-1,1",2,2",3,3",4,4"-octahydro-[1,1':3',1"-terphenyl]-2',4'-diol | | | |
| 7 | 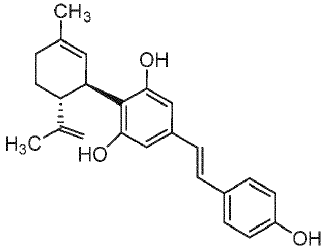 | increased (50% less than O1821) | | |
| | (1'R,2'R)-4-((E)-4-hydroxystyryl)-5'-methyl-2'-(prop-1-en-2-yl)-1',2',3',4'-tetrahydro-[1,1'-biphenyl]-2,6-diol | | | |
| 8 | 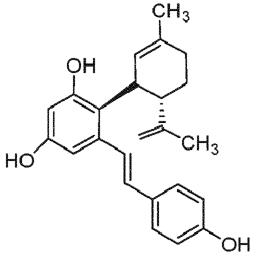 | increased (10% less than O1821) | | |
| | (1 'R,2'S)-6-((E)-4-hydroxystyryl)-5'-methyl-2 '-(prop-1-en-2-yl)-1',2',3',4'-tetrahydro-[1,1'-biphenyl]-2,4-diol | | | |
| 9 | 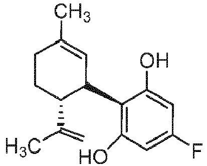 | increased (similar to O1821) | | |
| | (1'S,2'R)-4-fluoro-5'-methyl-2'-(prop-1-en-2-yl)-1',2',3',4'-tetrahydro-[1,1'-biphenyl]-2,6-diol | | | |
| 10 | 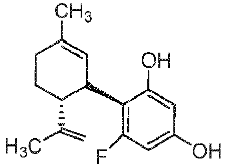 | increased (20% greater than 01821) | Decreased by 100% | Not protective KRN and CIA |
| | (1'S,2'R)-6-fluoro-5'-methyl-2'-(prop-1-en-2-yl)-1',2',3',4'-tetrahydro-[1,1'-biphenyl]-2,4-diol | | | |
| 11 | 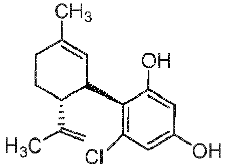 | increased (20% greater than O1821) | | |
| | (1'S,2'R)-6-chloro-5'-methyl-2'-(prop-1-en-2-yl)-1',2',3',4'-tetrahydro-[1,1'-biphenyl]-2,4-diol | | | |
| 12 | 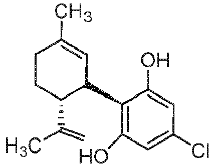 | increased greater than 01821) | Decreased by 100% | Not protective in CIA |
| | (1'S,2'R)-4-chloro-5'-methyl-2'-(prop-1-en-2-yl)-1',2',3',4'-tetrahydro-[1,1'-biphenyl]-2,6-diol | | | |
| 13 | 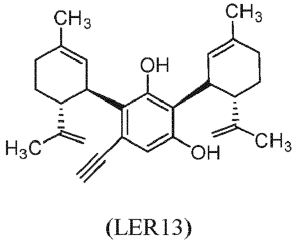 | increased (30% greater than O1821) | Decreased by 64% | Protective in CIA |
| | (1R,1"R,2R,2"S)-6'-ethynyl-5,5"-dimethyl-2,2"-di(prop-1-en-2-yl)-1,1",2,2",3,3",4,4"-octahydro-[1,1':3',1"-terphenyl]-2',4'-diol | | | |
| 14 | 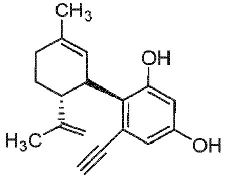 | increased greater than O1821) | Decreased by 100% | Not protective in KRN |
| | (1'S,2'R)-6-ethynyl-5'-methyl-2'-(prop-1-en-2-yl)-1',2',3',4'-tetrahydro-[1,1'-biphenyl]-2,4-diol | | | |

| | | | | |
|---|---|---|---|---|
| *Calcium influx measured over 200 seconds; FLS invasion evaluated over 24 hours in* in vitro *experiments; CIA=collagen-induced arthritis; KRN=KRN serum induced arthritis.* | | | | |

Treatment with LER13 significantly reduced arthritis severity and clinical signs of inflammation in DBA1/J mice with CIA (eight mice per group; FIG. 4B). The disease-protecting effect was detected by day 31 following the induction of CIA, and persisted though the end of the arthritis scoring period at day 76 (D76), with a reduction of 50% in mean cumulative arthritis scores, compared with control vehicle (arthritis severity index, ASI; mean±S.D.: control=80±21.8 and LER18 40.7±36.8; p=0.023, t-test; FIG. 4B). Maximum arthritis severity scores per mouse were also reduced by nearly 50% in LER13-treated mice compared with vehicle (mean±SD: LER13=7±4.2; vehicle control 13±2.44, p=0.004, t-test).

The number of joints with the maximum score (maximum score was 4 per paw per day and included avoidance of weight-bearing as a functional/disability component) was also examined during the 76 days of arthritis scoring as an estimate of the effect of LER13 at preventing the most severe and potentially disabling form of disease. The difference between vehicle-treated control and LER13 was significant at days 63 and 70 (D63: control=16 versus LER13=5, p=0.014; D70: control=12 versus LER13=1, p=0.001, Fisher's exact test).

The pronounced arthritis severity-improving effect observed with LER13 10 mg every-other-day in CIA, as opposed to 01821 10 mg BID *in vivo* (four-fold difference in daily dose) suggests that LER13 is more potent and has a longer half-life.

### Trpv2 Expression is Required for LER13-Induced Suppression

Trpv2 expression is required for the LER13-induced suppression of synovial cell invasion (FIG. 10). Cells treated with siRNA against Trpv2 are not responsive or suppressed by LER13.

### Cell Invasion Experiments with Two Different Cancer Cell Lines

Compound 01821 was tested in two different cancer cell lines (cervix carcinoma and glioblastoma) and was found to significantly reduce cell invasion (FIG. 9). *In vitro* cell invasion correlates with *in vivo* invasion and risk for metastasis. This is the first time that a Trpv2 agonist is shown to reduce cancer cell invasion.

### Example 17 - Discussion of Examples 1-16

While new and more effective therapies have been developed to treat RA, disease remission remains uncommon, and most patients only achieve modest to moderate reduction in disease activity. These observations underscore the need for more effective therapies. In these studies, efforts were focused on the identification and characterization of genes implicated in disease severity and joint damage as potential new targets for therapy. In a combination of genetic studies in rodent models of arthritis and functional and gene expression studies using FLS, a new role for the non-specific cation channel Trpv2 in the regulation of arthritis was discovered. The initial observation of increased expression of Trpv2 in highly invasive FLS, compared with minimally invasive FLS, suggested that the expression of this channel favors FLS invasion and joint damage. However, experiments with Trpv2 agonists established that this cation channel is in fact a suppressor of FLS invasion that reduces the expression of MMP-2 and MMP-3, two key proteases implicated in joint damage in RA and rodent models of arthritis. Knock-down of Trpv2 with siRNA also increased FLS invasion, further supporting its role in suppressing invasion.

The *in vitro* invasive properties of FLS have been shown to correlate with radiographic damage in RA, and with histologic damage and erosions in rodent models of arthritis (Tolboom et al., "Invasiveness of Fibroblast-Like Synoviocytes is an Individual Patient Characteristic Associated with the Rate of Joint Destruction in Patients with Rheumatoid Arthritis," Arthritis Rheum. 52(7): 1999-2002 (2005); Laragione et al., "The Arthritis Severity Locus Cia5d is a Novel Genetic Regulator of the Invasive Properties of Synovial Fibroblasts," Arthritis Rheum. 58(8):2296-2306 (2008), which are hereby incorporated by reference in their entirety). This *in vitro* model of invasion is also a highly useful screening method to identify relevant pathways and cellular processes for therapeutic targeting (Laragione et al., "mTOR Regulates the Invasive Properties of Synovial Fibroblasts in Rheumatoid Arthritis," Mol. Med. 16(9-10):352-358 (2010); Carlson et al., "Rapamycin, a Potential Disease-Modifying Antiarthritic Drug," J. Pharmacol. Exp. Ther. 266(2):1125-1138 (1993); Mohan et al., "Blockade of Chemokine Receptor CXCR3 Inhibits T Cell Recruitment to Inflamed Joints and Decreases the Severity of Adjuvant Arthritis," J. Immunol. 179(12):8463-8469 (2007); Cejka et al., "Mammalian Target of Rapamycin Signaling is Crucial for Joint Destruction in Experimental Arthritis and is Activated in Osteoclasts from Patients with Rheumatoid Arthritis," Arthritis Rheum. 62(8):2294-2302 (2010); Bruyn et al., "Everolimus in Patients with Rheumatoid Arthritis Receiving Concomitant Methotrexate: A 3-Month, Double-Blind, Randomised, Placebo-Controlled, Parallel-Group, Proof-Of-Concept Study," Ann. Rheum. Dis. 67(8):1090-1095 (2008); Yellin et al., "A Phase II, Randomized, Double-Blind, Placebo-Controlled Study Evaluating the Efficacy and Safety of MDX-1100, a Fully Human Anti-CXCL10 Monoclonal Antibody, in Combination with Methotrexate in Patients with Rheumatoid Arthritis," Arthritis Rheum. 64(6):1730-1739 (2012), which are hereby incorporated by reference in their entirety). Therefore, the commercially-available Trpv2-specific agonist 01821 was initially used. 01821 was effective at reducing invasiveness of FLS from patients with RA and FLS from DA rats with PIA by over 90%. 01821 was also effective at reducing mouse FLS invasion and clinical disease severity and histologic joint damage and pannus formation *in vivo* in the KRN serum transfer model where therapy was started after the onset of arthritis, thus mimicking the RA clinical setting.

While 01821 reduced disease severity *in vivo,* it required high-doses to obtain a modest effect. Therefore, the aim was to develop a new and more potent Trpv2 agonist. Twenty-one new compounds were developed and screened for Trpv2 agonistic activity based on their ability to increase calcium influx and inhibit FLS invasion. Based on those screening parameters, five different compounds were selected and tested *in vivo* in CIA in mice, a chronic and well-established classic model of RA. One of these five compounds, LER13, was significantly effective and reduced arthritis severity scores, number of joints with functional impairment, and the maximum arthritis scores by 50%. These studies are the first to use Trpv2 agonists *in vivo,* and no evidence of significant toxicities, mortality, infections, or tumors were observed, suggesting that these are promising new category of therapeutic agents.

To understand the processes regulated by Trpv2 in arthritis severity and FLS invasion several parameters were examined, including FLS morphology, the actin cytoskeleton, and the formation of lamellipodia, but none of those were affected by Trpv2 stimulation. However, the FLS expression of MMP-2 and MMP-3, two key mediators of invasion and joint damage, was significantly reduced by Trpv2 stimulation. These observations suggest that Trpv2 does not necessarily interfere with the FLS ability to move, but may instead affect invasion and joint damage via interference with an effector pathway such as MMP expression or activation. Thus, Trpv2 agonists, such as 01821 and LER13, may be useful to treat not only arthritis, but also other diseases such as cancers where cell invasion has a central role in pathogenesis and outcome.

That Trpv2 could be inhibiting MMP expression via interference with a signaling pathway implicated on MMP transcription or in cell invasion was considered. Akt, Erk, and NFκB were obvious candidates since they have been implicated in the regulation of cell invasion (Tobar et al., "ROS-NFkappaB Mediates TGF-Betal-Induced Expression of Urokinase-Type Plasminogen Activator, Matrix Metalloproteinase-9 and Cell Invasion," Mol. Cell Biochem. 340(1-2):195-202 (2010), which is hereby incorporated by reference in its entirety), and arthritis severity and joint damage (Miagkov et al., "NF-KappaB Activation Provides the Potential Link Between Inflammation and Hyperplasia in the Arthritic Joint," Proc. Natl. Acad. Sci. U.S.A. 95(23):13859-13864 (1998), which is hereby incorporated by reference in its entirety). Akt and Erk were previously shown to mediate Trpv2 activity in experiments unrelated to invasion with endothelial cells (Offertaler et al., "Selective Ligands and Cellular Effectors of a G Protein-Coupled Endothelial Cannabinoid Receptor," Mol. Pharmacol. 63(3):699-705 (2003), which is hereby incorporated by reference in its entirety) and glioma cells (Nabissi et al., "TRPV2 Channel Negatively Controls Glioma Cell Proliferation and Resistance to Fas-Induced Apoptosis in ERK-Dependent Manner," Carcinogenesis 31(5):794-803 (2010), which is hereby incorporated by reference in its entirety). Furthermore, the ankyrin domains in Trpv2 could potentially interact and interfere with NFκB activity (McCleverty et al., "Crystal Structure of the Human TRPV2 Channel Ankyrin Repeat Domain," Protein Sci. 15(9):2201-2206 (2006), which is hereby incorporated by reference in its entirety). However, no significant difference or reduction on levels of activation of these signaling proteins was detected. Several other signaling pathways could be involved in mediating the Trpv2-induced suppressive effect on FLS invasion and arthritis severity (Laragione et al., "mTOR Regulates the Invasive Properties of Synovial Fibroblasts in Rheumatoid Arthritis," Mol. Med. 16(9-10):352-358 (2010); Chan et al., "The GTPase Rac Regulates the Proliferation and Invasion of Fibroblast-Like Synoviocytes from Rheumatoid Arthritis Patients," Mol. Med. 13(5-6):297-304 (2007); Lin et al., "IL-6 Induces AGS Gastric Cancer Cell Invasion Via Activation of the c-Src/RhoA/ROCK Signaling Pathway," Int. J. Cancer 120(12):2600-2608 (2007); Li et al., "STAT3 Knockdown Reduces Pancreatic Cancer Cell Invasiveness and Matrix Metalloproteinase-7 Expression in Nude Mice," PLoS One 6(10):e25941 (2011); Price et al., "Regulation of the Cytoskeleton by Rho-Family GTPases: Implications for Tumour Cell Invasion," Semin. Cancer Biol. 11(2):167-173 (2001); Stengel et al., "Cdc42 in Oncogenic Transformation, Invasion, and Tumorigenesis," Cell Signal. 23(9):1415-1423 (2011); Kikuchi et al., "Invasion of Breast Cancer Cells into Collagen Matrix Requires TGF-Alpha and Cdc42 Signaling," FEBS Lett. 585(2):286-290 (2011); Friedl et al., "Cancer Invasion and the Microenvironment: Plasticity and Reciprocity," Cell 147(5):992-1009 (2011); Leivonen et al., "Transforming Growth Factor-Beta Signaling in Cancer Invasion and Metastasis," Int. J. Cancer 121(10):2119-2124 (2007), which are hereby incorporated by reference in their entirety).

Trpv2 stimulation also reduced the numbers of synovial-infiltrating inflammatory cells and numbers of synovial vessels. These observations suggest that Trpv2 could interfere with the ability of inflammatory cells or endothelial cell precursors to infiltrate the synovial tissue via a direct effect or through the suppression of chemotactic and angiogenic factors. Therefore, Trpv2 agonists may have a role in the treatment of other inflammatory and autoimmune diseases, as well as in diseases characterized by pronounced angiogenesis.

Although preferred embodiments have been depicted and described in detail herein, it will be apparent to those skilled in the relevant art that various modifications, additions, substitutions, and the like can be made without departing from the spirit of the invention and these are therefore considered to be within the scope of the invention as defined in the claims which follow.

## Claims

1. A compound of formula (Ia) 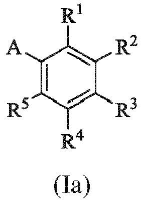 or a stereoisomer, pharmaceutically acceptable salt, oxide, solvate, or ester thereof, wherein
A is C₆₋₈ cycloalkenyl optionally substituted from 1 to 3 times with substituents independently selected from the group consisting of hydrogen, C₁₋₆ alkyl, and C₂₋₆ alkenyl;
R¹ is halogen, OH, C₁₋₆ alkyl, C₂₋₆ alkenyl, or C₂₋₆ alkynyl, wherein C₂₋₆ alkenyl is optionally substituted from 1 to 3 times with aryl, and wherein aryl is optionally substituted from 1 to 3 times with substituents independently selected from the group consisting of hydrogen, OH, and C₁₋₆ alkyl;
R² is hydrogen or C₆₋₈ cycloalkenyl, wherein C₆₋₈ cycloalkenyl is optionally substituted from 1 to 3 times with substituents independently selected from the group consisting of hydrogen, C₁₋₆ alkyl, and C₂₋₆ alkenyl;
R³ is halogen, OH, C₂₋₆ alkenyl, or C₂₋₆ alkynyl, wherein C₂₋₆ alkenyl is optionally substituted from 1 to 3 times with aryl, and wherein aryl is optionally substituted from 1 to 3 times with substituents independently selected from the group consisting of hydrogen, OH, and C₁₋₆ alkyl;
R⁴ is hydrogen or C₆₋₈ cycloalkenyl, wherein C₆₋₈ cycloalkenyl is optionally substituted from 1 to 3 times with substituents independently selected from the group consisting of hydrogen, C₁₋₆ alkyl, and C₂₋₆ alkenyl; and
R⁵ is halogen, OH, C₁₋₆ alkyl, C₂₋₆ alkenyl, or C₂₋₆ alkynyl, wherein C₂₋₆ alkenyl is optionally substituted from 1 to 3 times with aryl, and wherein aryl is optionally substituted from 1 to 3 times with substituents independently selected from the group consisting of hydrogen, OH, and C₁₋₆ alkyl,
for use in a method for the treatment of a disease or disorder associated with Trpv2 activity.

2. The compound for use according to claim 1, wherein the compound is a compound of formula (IIa) 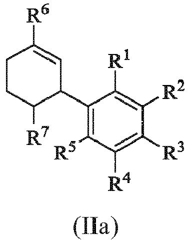 or a stereoisomer, pharmaceutically acceptable salt, oxide, solvate, or ester thereof, wherein
R¹ is halogen, OH, C₁₋₆ alkyl, C₂₋₆ alkynyl, or 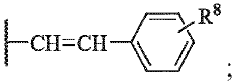
R² is hydrogen or 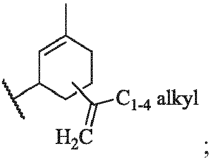
R³ is halogen, OH, 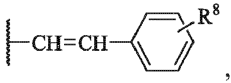 or C₂₋₆ alkynyl;
R⁴ is hydrogen or 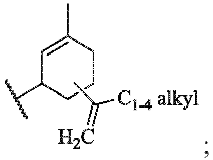
R⁵ is halogen, OH, C₁₋₆ alkyl, C₂₋₆ alkynyl, or 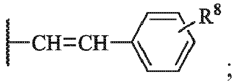
R⁶ is hydrogen or C₁₋₆ alkyl;
R⁷ is 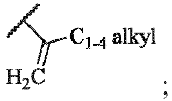 and
R⁸ is hydrogen, OH, or C₁₋₆ alkyl.

3. The compound for use according to claim 1 or claim 2, wherein in the compound of formula (IIa)
halogen is F or Cl;
C₁₋₆ alkyl is methyl;
C₁₋₄ alkyl is methyl;
C₂₋₆ alkynyl is C₂ alkynyl;
R⁷ is 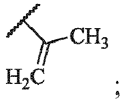 and
R⁸ is OH.

4. The compound for use according to claim 1, wherein the compound of formula (Ia) is selected from the group consisting of:

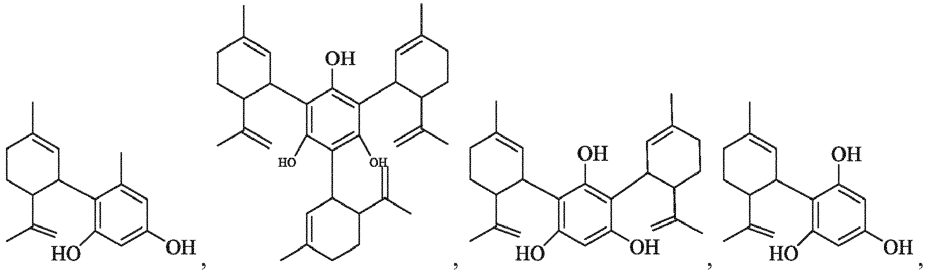

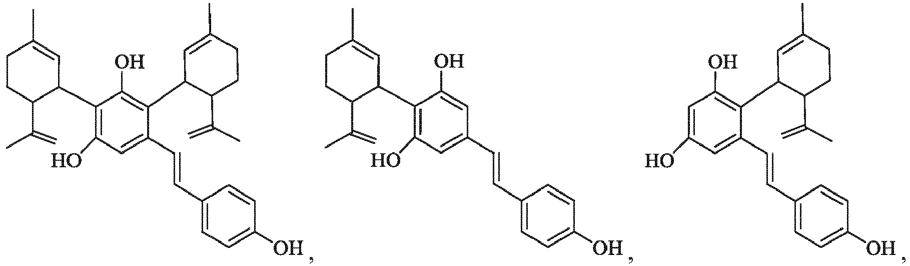

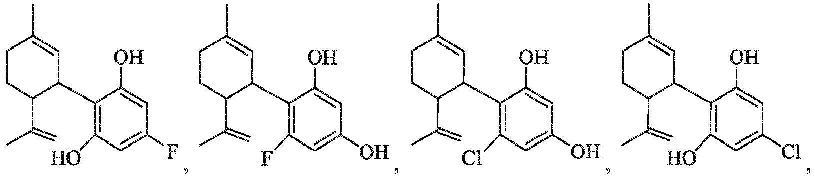

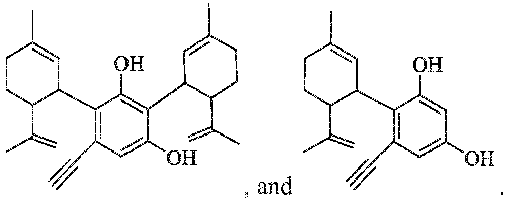

5. The compound for use according to claim 1, wherein the compound of formula (Ia) is

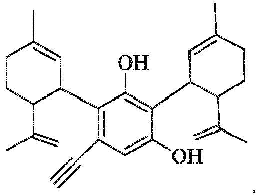

6. The compound for use according to any one of the preceding claims, wherein the disease or disorder associated with Trpv2 activity is selected from the group consisting of rheumatoid arthritis, psoriasis, psoriatic arthritis, inflammatory diseases, asthma, cancer, diabetic retinopathy, cardiomyopathy, heart failure, and congestive heart failure.

7. The compound for use according to any one of the preceding claims, wherein said administering is carried out orally, topically, transdermally, parenterally, subcutaneously, intravenously, intramuscularly, intraperitoneally, by intranasal instillation, by intracavitary or intravesical instillation, intraocularly, intraarterially, intralesionally, or by application to mucous membranes.

8. The compound for use according to any one of the preceding claims, wherein the subject is a mammal.

9. A compound of formula (Ib) 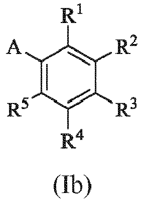 or a stereoisomer, pharmaceutically acceptable salt, oxide, solvate, or ester thereof, wherein
A is C₆₋₈ cycloalkenyl optionally substituted from 1 to 3 times with substituents independently selected from the group consisting of hydrogen, C₁₋₆ alkyl, and C₂₋₆ alkenyl;
R¹ is OH, C₂₋₆ alkenyl, or C₂₋₆ alkynyl, wherein C₂₋₆ alkenyl is optionally substituted from 1 to 3 times with aryl, and wherein aryl is optionally substituted from 1 to 3 times with substituents independently selected from the group consisting of hydrogen, OH, and C₁₋₆ alkyl;
R² is hydrogen or C₆₋₈ cycloalkenyl, wherein C₆₋₈ cycloalkenyl is optionally substituted from 1 to 3 times with substituents independently selected from the group consisting of hydrogen, C₁₋₆ alkyl, and C₂₋₆ alkenyl;
R³ is halogen, OH, C₂₋₆ alkenyl, or C₂₋₆ alkynyl, wherein C₂₋₆ alkenyl is optionally substituted from 1 to 3 times with aryl, and wherein aryl is optionally substituted from 1 to 3 times with substituents independently selected from the group consisting of hydrogen, OH, and C₁₋₆ alkyl;
R⁴ is hydrogen or C₆₋₈ cycloalkenyl, wherein C₆₋₈ cycloalkenyl is optionally substituted from 1 to 3 times with substituents independently selected from the group consisting of hydrogen, C₁₋₆ alkyl, and C₂₋₆ alkenyl; and
R⁵ is OH, C₂₋₆ alkenyl, and C₂₋₆ alkynyl, wherein C₂₋₆ alkenyl is optionally substituted from 1 to 3 times with aryl, and wherein aryl is optionally substituted from 1 to 3 times with substituents independently selected from the group consisting of hydrogen, OH, and C₁₋₆ alkyl;
with the proviso that (i) R³ cannot be F and (ii) when R¹, R³, and R⁵ are OH, R² and R⁴ cannot both be hydrogen.

10. The compound according to claim 9 having a structure of formula (IIb) 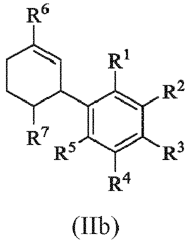 or a stereoisomer, pharmaceutically acceptable salt, oxide, solvate, or ester thereof, wherein
R¹ and R⁵ are independently OH, C₂₋₆ alkynyl, or 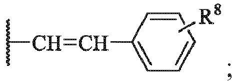
R² and R⁴ are independently hydrogen or 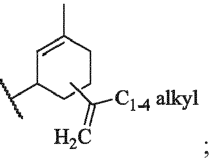
R³ is Cl, OH, 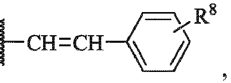 or C₂₋₆ alkynyl;
R⁶ is hydrogen or C₁₋₆ alkyl;
R⁷ is 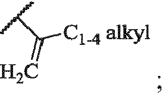 and
R⁸ is hydrogen, OH, or C₁₋₆ alkyl,
with the proviso that when R¹, R³, and R⁵ are OH, R² and R⁴ cannot both be hydrogen.

11. The compound according to claim 9 or claim 10, wherein C₂₋₆ alkynyl is C₂ alkynyl;
R⁷ is 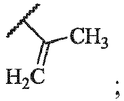 and
R⁸ is OH.

12. The compound according to claim 11, wherein the compound of formula (Ib) is selected from the group consisting of:

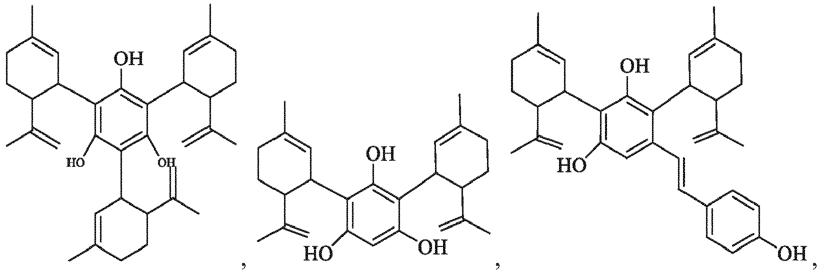

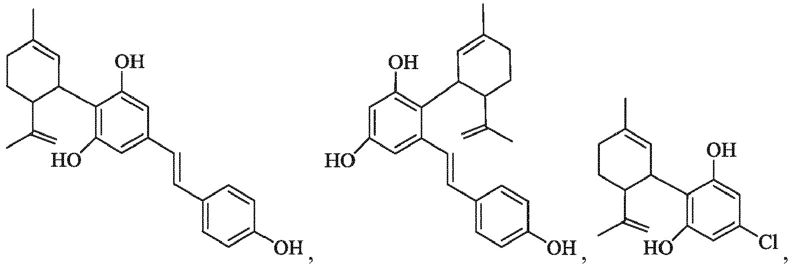

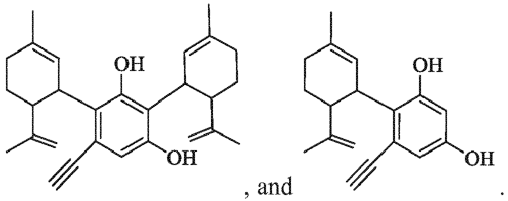

13. The compound according to claim 9, wherein the compound of formula (Ib) is

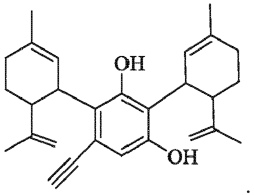

14. A composition comprising the compound according to any one of claims 9-13 and a carrier.

15. An *in vitro* method of increasing Trpv2 activity in a cell or tissue, said method comprising:
providing a compound as described in any one of claims 1-5; and
contacting a cell or tissue with the compound under conditions effective to increase Trpv2 activity in the cell or tissue.


SEQUENCE LISTING

<110>  The Feinstein Institute for Medical Research

<120>  TREATMENT METHOD, COMPOUNDS, AND METHOD OF INCREASING TRPV2
       ACTIVITY

<130>  29539.0561

<150>  62/126,167
<151>  2015-02-27

<150>  PCT/US2016/019776
<151>  2016-02-26

<160>  4

<170>  PatentIn version 3.5

<210>  1
<211>  20
<212>  DNA
<213>  Artificial Sequence

<220>
<223>  Primer

<400>  1
cggtttctc gaatccatga                                                    20


<210>  2
<211>  18
<212>  DNA
<213>  Artificial Sequence

<220>
<223>  Primer

<400>  2
ggtatccatc gccatgct                                                     18


<210>  3
<211>  19
<212>  DNA
<213>  Artificial Sequence

<220>
<223>  Primer

<400>  3
ccaggtgtgg agttcctga                                                    19


<210>  4
<211>  20
<212>  DNA
<213>  Artificial Sequence

<220>
<223>  Primer

<400>  4
gcatcttttg gcaaatctgg                                                    20